# EUROPEAN PATENT APPLICATION

(11) **EP 2 057 956 A1**
(43) Date of publication of application: **13.05.2009**
(21) Application number: 08019375.8
(22) Date of filing: 05.11.2008
(51) Int. Cl.: A61B 19/00

(54) **Endoscope washing and disinfecting apparatus**

(30) Priority: 12.11.2007 JP 2007293613; 27.12.2007 JP 2007337658
(71) Applicant: Olympus Medical Systems Corporation, Tokyo 151-0072 (JP)
(72) Inventor: Onishi, Hideto, Tokyo 151-0072 (JP)
(74) Representative: von Hellfeld, Axel

(57) **Abstract**

An endoscope washing and disinfecting apparatus (1) includes an apparatus main body (2) equipped with a washing bath (4) in which an endoscope (101) is mounted; a nozzle unit (70) provided so as to face a connector portion (104) of an endoscope conduit provided in the endoscope mounted in the washing bath, to which fluid within the apparatus main body is supplied so that the nozzle unit advances due to pressure of the fluid, and which supplies the fluid to inside the endoscope conduit by connecting to the connector portion; and an automatic brush mechanism (80) that is provided in the apparatus main body and that carries out brushing and cleaning within the endoscope conduit by introducing a cleaning brush (81) into the endoscope conduit through the nozzle unit and moving the cleaning brush (81) forward and rearward.

## Description

### BACKGROUND OF THE INVENTION

### 1. Field of the Invention

The present invention relates to an endoscope washing and disinfecting apparatus that carries out washing and disinfecting of an endoscope, and more particularly to an endoscope washing and disinfecting apparatus in which a nozzle that supplies a fluid to a channel of an endoscope is detachably mounted automatically to a connector portion of the channel.

### 2. Description of the Related Art

In recent years, endoscopes are widely utilized in the fields of medical and manufacturing industry. Medical endoscopes have an elongated insertion portion that is inserted into a body cavity to enable observation of an organ within the body cavity or, as necessary, to enable various kinds of treatment using treatment instruments that are inserted into a treatment instrument insertion channel.

In particular, since endoscopes used in the medical treatment field are inserted into a body cavity and used for the purpose of endoscopy and treatment, it is necessary to clean and disinfect the endoscopes. An endoscope washing and disinfecting apparatus is used when an endoscope is cleaned and disinfected. The endoscope is placed inside a washing bath of the endoscope washing and disinfecting apparatus and then washed, disinfected, rinsed out, and drained.

The inside of an endoscope has a plurality of conduits such as an air supply/water supply conduit and a forceps opening. It is necessary that a sufficient quantity of cleaning fluid and disinfectant fluid passes through the inside of these conduits, i.e. channels, to ensure cleaning and disinfecting are reliably performed.

As a tool that cleans inside the channels of an endoscope in this manner, for example, Japanese Patent Application Laid-Open Publication No. 2002-200031 discloses an auxiliary cleaning tool that is capable of selectively inserting a cleaning brush into a plurality of conduits of an endoscope. Further, Japanese Patent Application Laid-Open Publication No. 2006-55325 discloses a coupler which enables a tubular member for guiding a cleaning brush or the like into a channel to be easily attached to an endoscope.

Furthermore, a device is disclosed, for example, in Japanese Patent Application Laid-Open Publication No. 11-99121 as an endoscope washing and disinfecting apparatus (endoscope cleaning apparatus) that washes and disinfects an endoscope and conduits (channels) inside the endoscope.

The conventional endoscope washing and disinfecting apparatus includes a cleaning liquid supply nozzle device. The cleaning liquid supply nozzle device includes a nozzle that can be detachably mounted on the same axis as an air supply/water supply connector provided in an LG connector serving as an opening of a conduit inside the endoscope. The cleaning liquid supply nozzle device is provided with a mounting/demounting mechanism so that, during cleaning, the nozzle separates from the air supply/water supply connector with which the nozzle is in contact, to thereby enable the section at which the air supply/water supply connector and the nozzle are in contact at the time of connection to be cleaned.

However, according to the technology disclosed in Japanese Patent Application Laid-Open Publication No. 2002-200031 and Japanese Patent Application Laid-Open Publication No. 2006-55325, it is necessary for the user to manually attach the auxiliary cleaning tool or the coupler to a base that constitutes an opening of each channel of the endoscope. Therefore, the user must confirm whether or not the auxiliary cleaning tool or the coupler is correctly connected to the endoscope.

Furthermore, for hygienic reasons, since it is necessary to carry out brushing and cleaning using a cleaning brush for each channel of an endoscope after use thereof, there is a problem that the user is forced to carry out extremely troublesome work.

Moreover, in some endoscopes the number of channels provided and the positions at which the bases of the channels are located differ depending on the model. Consequently, in particular in relation to the coupler disclosed in Japanese Patent Application Laid-Open Publication No. 2006-55325, there has been a problem that a corresponding device must be prepared for each endoscope model.

Furthermore, the cleaning liquid supply nozzle device disclosed in Japanese Patent Application Laid-Open Publication No. 11-99121 is arranged so that a nozzle can be detachably mounted on the same axis as an air supply/water supply connector of the endoscope. Therefore, depending on the shape of the air supply/water supply connector, it is difficult for a stream of cleaning fluid to flow over the entire outer surface of the air supply/water supply connector and the root portion of the air supply/water supply connector. Further, for this kind of conventional endoscope washing and disinfecting apparatus it has been necessary to set a long washing time in order to reliably clean the entire outer surface of the air supply/water supply connector as well as the root portion thereof.

The present invention was made in consideration of the above described problems, and an object of the present invention is to provide an endoscope washing and disinfecting apparatus that can be detachably mounted automatically to a channel of an endoscope in a manner corresponding to the endoscope model, and that can carry out washing and disinfecting by supplying fluid such as a cleaning fluid and a disinfectant fluid and also automatically carry out brushing and cleaning within a channel.

A further object of the present invention is to provide an endoscope washing and disinfecting apparatus in which a nozzle for supplying a fluid such as a cleaning fluid can be advanced or withdrawn so as to be detachably mounted to a conduit of an endoscope so that an outer surface portion and a root portion of a connector portion at an opening of a conduit of the endoscope in question can be reliably cleaned in a short time.

### SUMMARY OF THE INVENTION

An endoscope washing and disinfecting apparatus of the present invention includes: an apparatus main body that includes a washing bath in which an endoscope is placed; a nozzle unit that is arranged so as to face a connector portion of an endoscope conduit that is provided in the endoscope that is placed in the washing bath, to which fluid within the apparatus main body is supplied so that the nozzle unit advances due to pressure of the fluid, and which supplies the fluid to inside of the endoscope conduit by connecting to the connector portion; and an automatic brush mechanism that is arranged in the apparatus main body and that carries out brushing and cleaning within the endoscope conduit by introducing a cleaning brush into the endoscope conduit through the nozzle unit and moving the cleaning brush forward and rearward.

The above and other objects, features and advantages of the invention will become more clearly understood from the following description referring to the accompanying drawings.

### BRIEF DESCRIPTION OF THE DRAWINGS

Fig. 1 is an oblique perspective view of an endoscope washing and disinfecting apparatus according to a first embodiment of the present invention;
Fig. 2 is a configuration view that schematically illustrates the configuration of the endoscope washing and disinfecting apparatus according to the first embodiment;
Fig. 3 is an oblique perspective view showing the configuration of a conduit connection unit according to the first embodiment;
Fig. 4 is a plan view of the conduit connection unit shown in Fig. 3 as viewed from the direction of arrow IV;
Fig. 5 is an exploded oblique perspective view illustrating a nozzle unit that is housed in a rotary cylinder according to the first embodiment;
Fig. 6 is an oblique perspective view for describing the configuration of a rotational position detecting switch that detects a rotational position of the rotary cylinder according to the first embodiment;
Fig. 7 is a cross-sectional view of the conduit connection unit that is disposed in a washing bath according to the first embodiment;
Fig. 8 is a view that illustrates movement positions of a conduit connector of the nozzle unit upon rotation of the rotary cylinder according to the first embodiment;
Fig. 9 is a view for describing actions of the conduit connection unit when an endoscope has two channel connector portions according to the first embodiment;
Fig. 10 is a view for describing actions of the conduit connection unit when an endoscope has one channel connector portion according to the first embodiment;
Fig. 11 is an oblique perspective view showing the configuration of a conduit connection unit according to a second embodiment of the present invention;
Fig. 12 is a cross-sectional view of the conduit connection unit according to the second embodiment;
Fig. 13 is a view for describing a state in which, according to the second embodiment, in response to rotation of an outer rotary body, an inner rotation side gear of an inner rotary body meshes with a frame body side gear of a frame body, and as a result the inner rotary body rotates around a central axis;
Fig. 14 is a view for describing a state in which, according to the second embodiment, the outer rotary body makes a positive rotation by 90° from the state shown in Fig. 13, and the inner rotary body counter-rotates by 90° around the central axis;
Fig. 15 is a view for describing a state in which, according to the second embodiment, the outer rotary body makes a positive rotation by 90° from the state shown in Fig. 14, and the inner rotary body counter-rotates by 90° around the central axis;
Fig. 16 is a view for describing a state in which, according to the second embodiment, a conduit connector of a nozzle unit of the inner rotary body moves due to rotation of the inner rotary body around the central axis;
Fig. 17 is a view for describing a position to which the conduit connector of the nozzle unit moves when the outer rotary body rotates by 90° from the state shown in Fig. 16 according to the second embodiment;
Fig. 18 is a view for describing a position to which the conduit connector of the nozzle unit moves when the outer rotary body rotates by 90° from the state shown in Fig. 17 according to the second embodiment;
Fig. 19 is a view for describing actions of a conduit connection unit when an endoscope has two channel connector portions according to the second embodiment;
Fig. 20 is a view for describing actions of the conduit connection unit when an endoscope has one channel connector portion according to the second embodiment;
Fig. 21 is a partial cross-sectional view that illustrates an automatic brush mechanism that is moved in two directions by a motor, and a configuration in which a nozzle unit is provided in the automatic brush mechanism, according to a third embodiment of the present invention;
Fig. 22 is a partial cross-sectional view for describing a conduit switching mechanism portion that switches flow channels which is inserted into two nozzle units according to a fourth embodiment of the present invention;
Fig. 23 is a cross-sectional view that illustrates a forceps plug that is detachably mounted to two channel connector portions arranged in an operation portion of an endoscope according to a fifth embodiment of the present invention;
Fig. 24 is an oblique perspective view that illustrates the configuration of a conduit connection unit according to a sixth embodiment of the present invention;
Fig. 25 is a plan view of the conduit connection unit shown in Fig. 24 as viewed from the direction of arrow XXV in Fig. 24;
Fig. 26 is a cross-sectional view of the conduit connection unit disposed in a washing bath according to the sixth embodiment;
Fig. 27 is a view for describing a plurality of positions at which a nozzle unit is connected to a channel connector portion of the endoscope according to the sixth embodiment;
Fig. 28 is a view for describing a plurality of positions at which a nozzle unit abuts against and is regulated by a nozzle abutment portion according to the sixth embodiment;
Fig. 29 is a front view of the conduit connection unit for describing an action through which the nozzle unit is connected to the channel connector portion of an endoscope according to the sixth embodiment;
Fig. 30 is a cross-sectional view of the conduit connection unit taken along line XXX-XXX in Fig. 29;
Fig. 31 is a front view of the conduit connection unit for describing an action at two positions at which the nozzle unit abuts against and is regulated by the nozzle abutment portion according to the sixth embodiment;
Fig. 32 is a cross-sectional view of the conduit connection unit taken along line XXXII-XXXII shown in Fig. 31 according to the sixth embodiment;
Fig. 33 is a front view of the conduit connection unit for describing, in correspondence to an endoscope including two channel connector portions, a state in which the nozzle unit is connected to one of the channel connector portions and in which the nozzle unit abuts against and is regulated by the nozzle abutment portion according to the sixth embodiment; and
Fig. 34 is a front view of the conduit connection unit for describing, in correspondence to an endoscope including two channel connector portions, a state in which the nozzle unit is connected to the other channel connector portion and in which the nozzle unit abuts against and is regulated by the nozzle abutment portion according to the sixth embodiment.

### DETAILED DESCRIPTION OF THE PREFERRED EMBODIMENTS

Hereunder, an endoscope washing and disinfecting apparatus of the present invention is described based on the attached drawings.

### (First Embodiment)

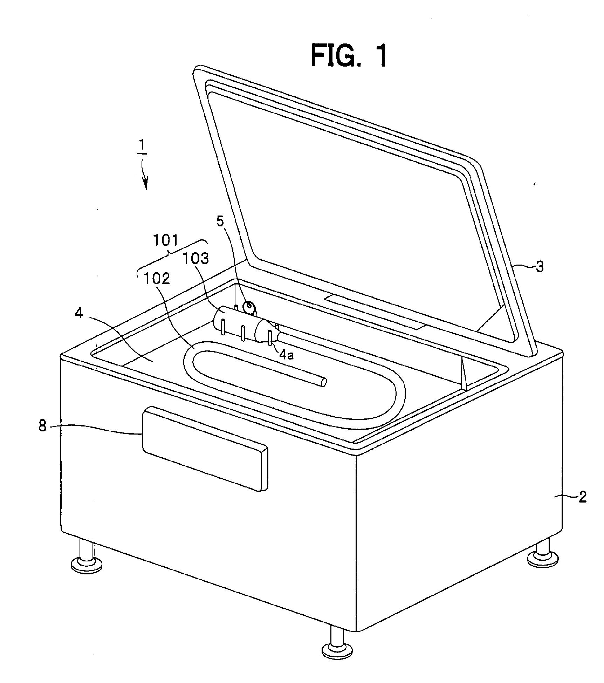

First, an endoscope washing and disinfecting apparatus according to a first embodiment of the present invention is described based on Fig. 1 to Fig. 10.

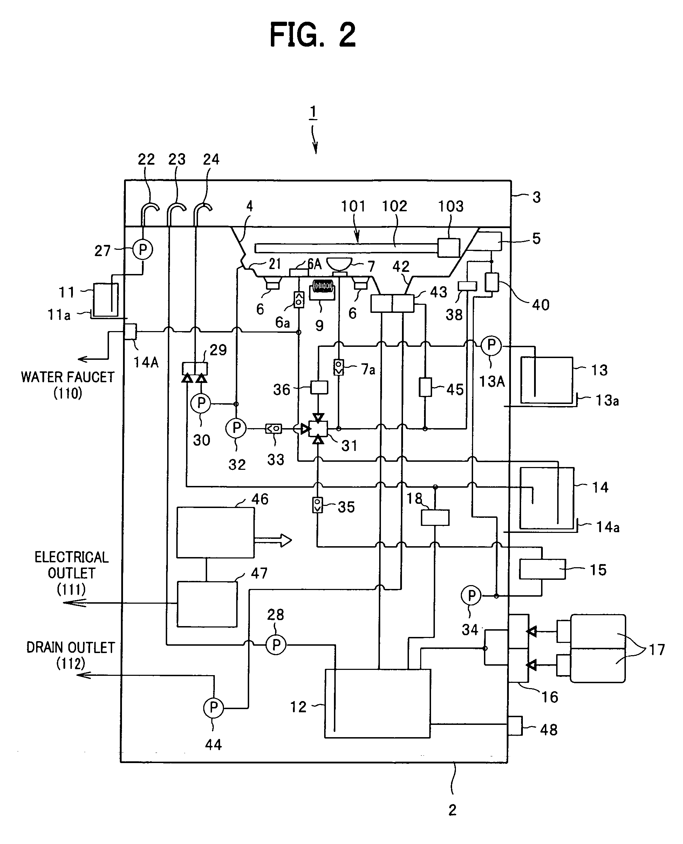

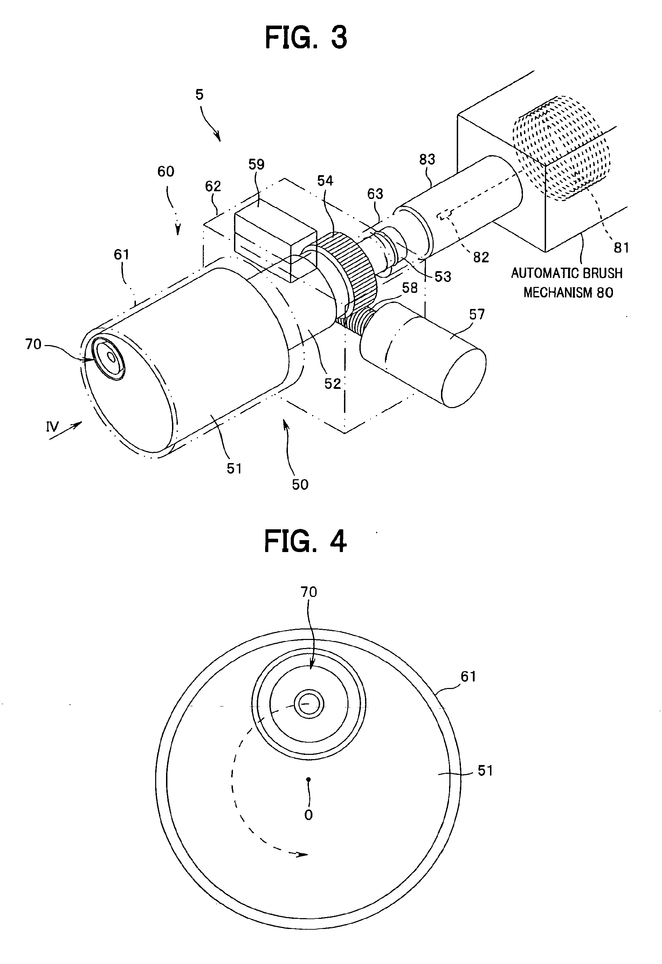

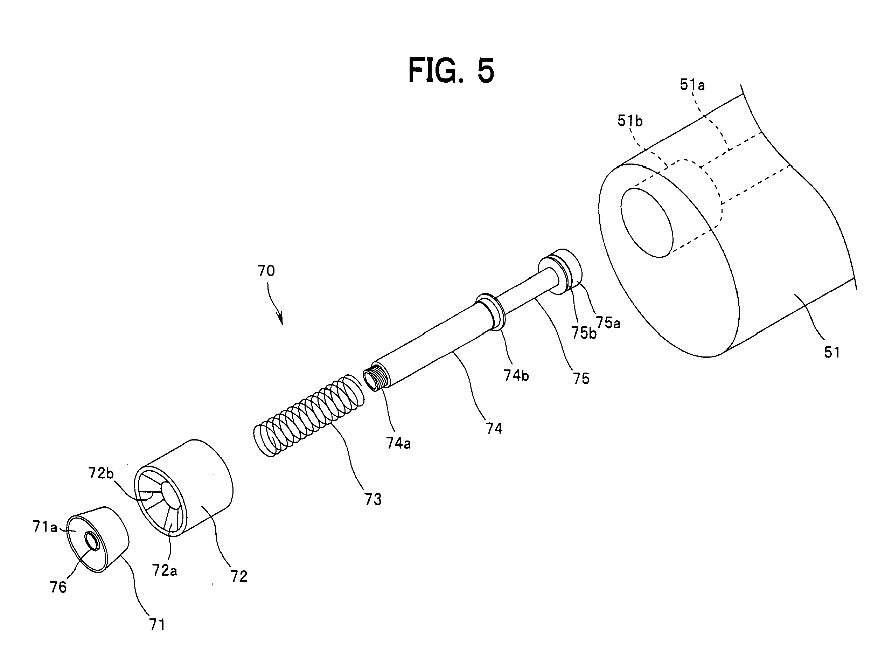

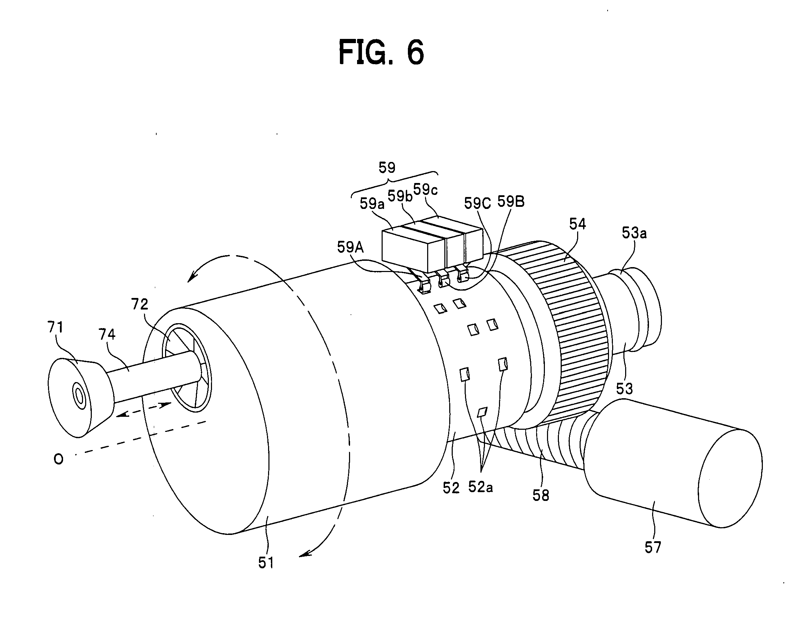

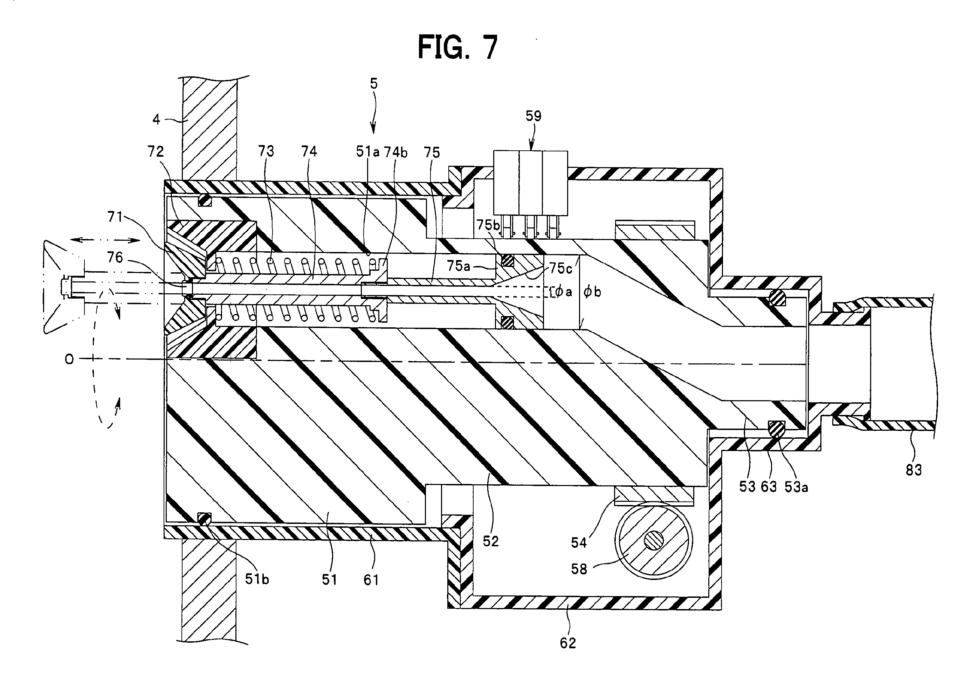

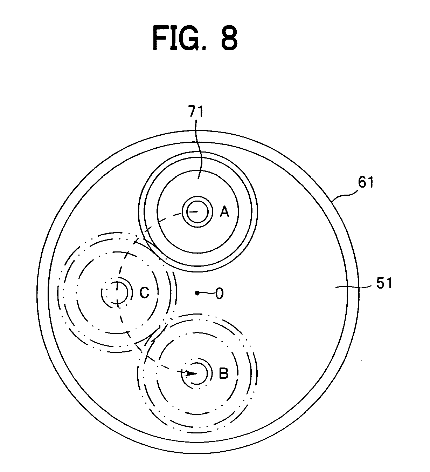

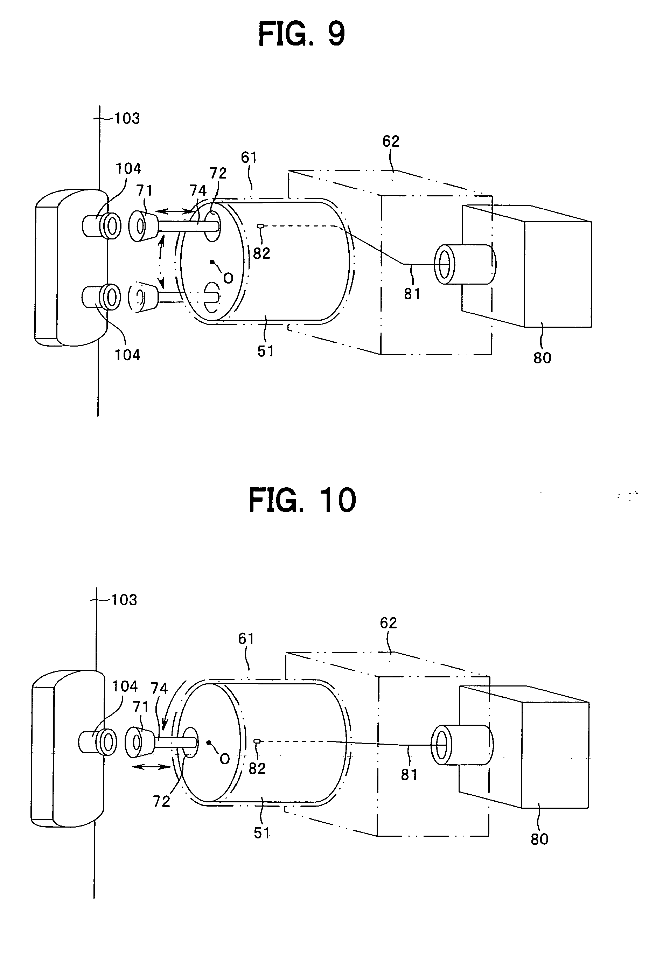

Fig. 1 to Fig. 10 relate to the first embodiment of the endoscope washing and disinfecting apparatus of the present invention. Fig. 1 is an oblique perspective view of an endoscope washing and disinfecting apparatus. Fig. 2 is a configuration diagram that schematically shows the configuration of the endoscope washing and disinfecting apparatus. Fig. 3 is an oblique perspective view illustrating the configuration of the conduit connection unit. Fig. 4 is a plan view of the conduit connection unit shown in Fig. 3 as viewed from the direction of arrow IV. Fig. 5 is an exploded oblique perspective view illustrating a nozzle unit that is housed in a rotary cylinder. Fig. 6 is an oblique perspective view for describing the configuration of a rotational position detecting switch that detects a rotational position of the rotary cylinder. Fig. 7 is a cross-sectional view of the conduit connection unit that is disposed in a washing bath. Fig. 8 is a view that illustrates movement positions of a conduit connector of the nozzle unit upon rotation of the rotary cylinder. Fig. 9 is a view for describing the action of the conduit connection unit when an endoscope has two channel connector portions. Fig. 10 is a view for describing the action of the conduit connection unit when an endoscope has one channel connector portion.

As shown in Fig. 1, an endoscope washing and disinfecting apparatus 1 has an apparatus main body 2 that is formed in a substantially cuboid shape overall and a top cover 3 that covers the top surface of the apparatus main body 2. The top cover 3 as a washing bath cover is attached to the top surface of the apparatus main body 2 by a hinge mechanism (not shown) in a condition in which the top cover 3 is capable of opening and closing.

A washing and disinfecting bath (hereunder, referred to as "washing bath") 4 capable of housing an endoscope 101 is provided at the top surface of the apparatus main body 2. In a state in which the top cover 3 is closed so as to cover the washing bath 4 of the apparatus main body 2, the endoscope 101 stored inside the washing bath 4 is washed and disinfected according to a predetermined washing and disinfecting process. The front surface of the apparatus main body 2 has an operation panel 8 that enables setting of start, stop, and various functions and also includes various display functions.

The endoscope 101 includes a flexible insertion portion 102 and an operation portion 103. An unshown channel as an endoscope conduit is provided in the insertion portion 102. The channel is an endoscope conduit for inserting a treatment instrument or the like and performing suction, supplying air, supplying water and the like.

The insertion portion 102 is bent and housed inside the washing bath 4. More specifically, the operation portion 103 is positioned and placed between a plurality of pins 4a provided inside the washing bath 4. A conduit connection unit 5 is arranged inside the apparatus main body 2 so as to be exposed on a wall surface of the washing bath 4 in the vicinity of the operation portion 103 that is positioned and housed in the washing bath 4. In this connection, a plurality of pins for positioning the insertion portion 102 in a predetermined shape may also be provided in the washing bath 4.

The conduit connection unit 5 has a mechanism for automatically connecting a connecting pipe to which a cleaning fluid or the like is supplied to a channel opening of the endoscope 101. A channel connector portion is provided in a channel opening that is described later, and the channel opening serves as the opening of various channels of the endoscope 101 that are provided in the operation portion 103. A nozzle that is described later is connected to the channel connector portion, fluid such as cleaning fluid is supplied to the nozzle, and a cleaning brush that is described later moves forward and backward at the nozzle. The configuration of the conduit connection unit 5 is described later.

As shown in Fig. 2, a plurality of ultrasonic transducers 6 are mounted on a bottom portion of the washing bath 4 that is provided on the top surface of the apparatus main body 2. The ultrasonic transducers 6 serve as vibration generating means that impart ultrasonic vibrations to the fluid during washing and disinfecting of the endoscope 101. A washing case 7 is provided inside the washing bath 4 at a substantially center region in which the endoscope 101 is not disposed. The washing case 7 is disposed for accommodating various buttons and the like that are removably attached to the endoscope 101. On the bottom portion of the washing bath 4 is provided a heater 9 for heating fluid inside the washing bath 4.

Inside the apparatus main body 2 are provided a detergent tank 11 that stores a liquid detergent, a disinfectant fluid tank 12 that stores a disinfectant fluid that is diluted to a predetermined concentration, an alcohol tank 13 that stores alcohol, a water filter 14 that filters tap water that is supplied from a water faucet 110, and an air filter 15. The disinfectant fluid tank 12 is fixed inside the apparatus main body 2, and the detergent tank 11, the alcohol tank 13, the water filter 14, and the air filter 15 are mounted on trays 11a and 13a to 15a, respectively.

By opening an unshown front surface door of the apparatus main body 2, each of the trays 11a, and 13a to 15a can be drawn out to the front to enable refilling of a predetermined fluid or replacement of a component part. The water faucet 110 is connected via a check valve 6a to a water supply-conduit disinfecting connector 6A that is provided at the bottom 4A of the washing bath 4.

Meanwhile since the disinfectant fluid tank 12 is fixedly arranged in the apparatus main body 2, refilling the disinfectant fluid to the disinfectant fluid tank 12 is performed by opening the front door of the apparatus main body 2 and connecting a disinfectant fluid bottle 17 filled with the disinfectant fluid to a bottle connector 16 fixed within the apparatus main body. Further, at that time, the tap water filtered by the water filter 14 is supplied to the disinfectant fluid tank 12 via a dilution valve 18. The tap water from the water faucet 110 is fed into the water filter 14 by opening a valve inside a water-supply valve 14A. Thus, a disinfectant fluid that is diluted to a predetermined concentration is stored in the disinfectant fluid tank 12. Fig. 2 illustrates a state in which the trays 11a and 13a to 15a are drawn out from the apparatus main body.

The washing bath 4 is provided with a detergent nozzle 22, a disinfectant fluid nozzle 23, and a water supply/circulation nozzle 24, which are located at a corner on an upper part of the washing bath 4. The detergent nozzle 22 communicates with the detergent tank 11 via a detergent pump 27, while the disinfectant fluid nozzle 23 communicates with the disinfectant fluid tank 12 via a chemical agent pump 28. Further, the water supply/circulation nozzle 24 communicates selectively with the water filter 14 or a liquid-supply pump 30 via a three-way valve 29. In a state in which the water supply/circulation nozzle 24 is connected to the water filter 14 side via the three-way valve 29, the tap water filtered by the water filter 14 is discharged from the water supply/circulation nozzle 24.

In contrast, in a state where the three-way valve 29 is switched to connect the water supply/circulation nozzle 24 to a flow pump 30, the washing water or disinfecting water pooled in the washing bath 4 is discharged and circulated via a circulation outlet 21 that is provided in an outer peripheral wall surface of a housing recess. Although not shown in Fig. 2, a high pressure nozzle is connected via a high pressure pump between the water supply/circulation nozzle 24 and the three-way valve 29. Fluids (such as tap water and washing water) that are similar to those from the water supply/circulation nozzle 24 are also discharged at high pressure from the high pressure nozzle.

The liquids discharged from the high pressure nozzle and water supply/circulation nozzle 24 generate a flow in the housing recess of the washing bath 4. This flow makes it possible to wash and rinse the outer surface of the endoscope 101. An unshown drain outlet is also provided at the bottom of the accommodation recess of the washing bath 4.

The circulation outlet 21 communicates with a CH (channel) block 31 serving as a four-way valve. In this communication passage, a CH (channel) pump 32 and a check valve 33 are arranged in the order from the circulation outlet 21. The check valve 33 is provided to prevent the washing water or the disinfecting water flowing to the CH pump 32 side. By driving the CH pump 32, the washing water or disinfecting water pooled in the housing recess is supplied to the channel block 31 side. The CH block 31 also communicates with the air filter 15 via an air check valve 35. The air check valve 35 is configured so that fluid (tap water, washing water, and disinfecting water) does not flow to the air filter 15 side. The air filter 15 communicates with a compressor 34, and compressed air from the compressor 34 is discharged to the CH block 31 side via the air filter 15.

Furthermore, the CH block 31 also communicates with the alcohol tank 13, and an alcohol pump 13A and an alcohol valve 36 are provided midway along the communication passage from the alcohol tank 13 side. The alcohol in the alcohol tank 13 is supplied by the alcohol pump 13A to the CH block 31 via the alcohol valve 36.

The above described fluids (tap water, washing water, and disinfecting water) that are supplied to the CH block 31 are fed to the conduit connection unit 5 provided in the side wall of the washing bath 4 via the CH block 31 and a CH (channel) valve 38 for each washing and disinfecting process. Air is also supplied to the conduit connection unit 5 from a compressor 34 via a pressure valve 40.

A drain outlet 42 is arranged in the bottom of the housing recess of the washing bath 4, and this drain outlet 42 is provided with a switching valve 43 that communicates with the disinfectant fluid tank 12. Selectively switching this valve 43 makes it possible to return the disinfectant fluid pooled in the housing recess of the washing bath 4 to the disinfectant fluid tank 12. The switching valve 43 also communicates with an external drain outlet 112 via a drain pipe 44a, so that fluids such as washing water and rinsing water are discharged to outside. Midway between the switching valve 43 and the drain pipe 44a is provided a drainage pump 44 for sucking in washing water and rinsing water that is pooled in the housing recess of the washing bath 4 and discharging the sucked-in fluid to outside. The drain outlet 42 of the washing bath 4 also communicates with the CH block 31 through a passage which is equipped with a bypass valve 45 midway to the block 31.

The disinfectant fluid stored in the disinfectant fluid tank 12, which is diluted to a predetermined concentration, is replaced with new fluid after every predetermined number of disinfecting processes. When the disinfectant fluid is replaced, the disinfectant fluid tank 12 communicates with a disinfectant fluid drain outlet 48 so that the disinfectant fluid is discharged to outside from the disinfectant fluid drain outlet 48.

A control portion 46 is provided within the apparatus main body 2 for controlling the aforementioned various pumps, valves and devices for each process. The control portion 46 as a controller is supplied with electric power through an electric cable from an external electrical outlet 111 via a power supply 47.

Next, referring to Figs. 3 to 7, the configuration of the conduit connection unit 5 that is arranged in the apparatus main body 2 so that a distal end portion thereof is exposed in the washing bath 4 is described in detail.

As shown in Fig. 3, the conduit connection unit 5 has a rotary cylinder 50 that is a rotating member in which a nozzle unit 70 (see Fig. 5) that is described later as a washing nozzle is housed in a condition in which the nozzle unit 70 can move forward and rearward, and a frame body 60 in which the rotary cylinder 50 is rotatably housed. Further, an automatic brush mechanism 80 is connected to the conduit connection unit 5.

The rotary cylinder 50 mainly includes, in order from the distal end thereof, a nozzle housing 51 that houses the nozzle unit 70 at a position that is decentered from the center of the nozzle housing 51, a rotational position detecting portion 52, and a rotation supporting portion 53. Each of these components constituting the rotary cylinder 50 is integrally formed in an externally cylindrical shape so as to have the same central axis O.

The rotary cylinder 50 also has a spur gear-shaped worm wheel 54 at an outer circumferential portion between the rotational position detecting portion 52 and the rotation supporting portion 53.

The frame body 60 has a distal end frame 61 that rotationally supports and houses the nozzle housing 51, and a proximal end frame 62 that is fitted and fixed at the rear side of the distal end frame 61 and that houses the rotational position detecting portion 52 and the worm wheel 54. Further, a tube connection portion 63 is provided in the proximal end frame 62 in a condition extending rearward therefrom. The tube connection portion 63 is connected with a fluid supply tube 83, and rotatably supports and houses the rotation supporting portion 53 of the rotary cylinder 50.

In this connection, the fluid supply tube 83 connects with the automatic brush mechanism 80. The automatic brush mechanism 80 is connected to a conduit that supplies fluid that is fed via the CH (channel) valve 38 and the pressure valve 40 shown in Fig. 2 to the conduit connection unit 5.

In the proximal end frame 62 are arranged a motor 57 as a driving source that includes a worm gear 58 that meshes with the worm wheel 54 and which has an axis of rotation that is perpendicular to but not intersecting with the axis of rotation (central axis O of the rotary cylinder 50) of the worm wheel 54, and a rotational position detecting switch 59 such as a limit switch at a position along the outer circumferential surface of the rotational position detecting portion 52.

More specifically, the rotary cylinder 50 is configured so that the driving force of the motor 57 is transmitted to the worm wheel 54 via the worm gear 58, and as shown in Fig. 4, the rotary cylinder 50 rotates around its central axis O and the rotational position thereof is detected by the rotational position detecting switch 59. The central axis O of the rotary cylinder 50 is the axis of rotation. Further, in the rotary cylinder 50, a front face portion of the nozzle housing 51 is rotatably supported so as to be exposed at an opening of the distal end frame 61.

An exchangeable cassette-type cleaning brush 81 is wound and housed in the automatic brush mechanism 80. A brush portion 82 is provided at the distal end of the cleaning brush 81. By means of a forward/rearward movement device composed from an unshown roller and motor and the like provided in the automatic brush mechanism 80, the cleaning brush 81 is moved forward and rearward to be led out from and introduced back into the automatic brush mechanism 80.

The cleaning brush 81 that is led out from the automatic brush mechanism 80 passes through the inside of the nozzle unit 70 and moves forward and rearward within the channel of the endoscope 101 to carry out brushing and cleaning. In this connection, the automatic brush mechanism 80 has a configuration that is used conventionally, and hence a description of the detailed configuration and action thereof is omitted here.

Next, referring to Fig. 5, the configuration of the nozzle unit 70 that is housed in the nozzle housing 51 is described in detail.

The nozzle unit 70 mainly includes a conduit connector 71, a base 72, a spring 73, a first nozzle pipe 74, and a second nozzle pipe 75.

The conduit connector 71 is a non-metal component that is made of synthetic resin or the like, and is a substantially cylindrical member with a so-called mortar-like tapered surface 71 a formed at the front thereof. A through-hole is formed along the axial direction of the conduit connector 71 at the center thereof. A gasket 76 that is formed of an elastic member in a substantially cylindrical shape is arranged in the through-hole so as to protrude at the front side of the conduit connector 71.

The base 72 is formed in a substantially annular shape, and has a recess 72a that houses the conduit connector 71 at a front portion thereof. A plurality of grooves 72b for allowing liquid to flow out are formed towards the outer circumference in the recess 72a. Further, in a state in which the nozzle unit 70 is assembled, the rear side of the base 72 contacts against a distal end portion of the spring 73.

The first nozzle pipe 74 is a metal tube, and has a screw portion 74a that is screwed with the proximal end face of the conduit connector 71 at the distal end thereof, and a flange 74b at the proximal end thereof that contacts against a proximal end portion of the spring 73 and receives an urging force therefrom. That is, in the nozzle unit 70, the first nozzle pipe 74 is passed through the spring 73 and the base 72 in that order, and the screw portion 74a and the conduit connector 71 are screwed together.

Further, a second nozzle pipe 75 that is made of metal is screwed to the proximal end of the first nozzle pipe 74. At the proximal end of the second nozzle pipe 75 is provided a resistor 75a with a substantially cylindrical external diameter for receiving the pressure of fluid that flows inside the nozzle housing 51. A circumferential groove is formed along the outer circumference of the resistor 75a, and an O-ring 75b is provided on the circumferential groove. The detailed configuration of the resistor 75a is described later.

The nozzle unit 70 configured in this manner is insertingly disposed in a nozzle insertion hole portion 51 a formed in the nozzle housing 51. In this connection, the base 72 of the nozzle unit 70 is fixed by adhesive bonding or the like to a hole portion that opens at the distal end face of the nozzle housing 51.

Further, the nozzle insertion hole portion 51 a is formed at a position that is decentered towards the outer circumferential side with respect to the center of the nozzle housing 51, and has substantially the same hole diameter as the outer diameter (bore) of the resistor 75a of the second nozzle pipe 75.

Next, referring to Fig. 6, a configuration that detects a rotating position around the central axis O of the rotary cylinder 50 using the rotational position detecting switch 59 is described in detail below.

As shown in Fig. 6, the rotational position detecting switch 59 includes three limit switches 59a to 59c. The rotational position detecting portion 52 of the rotary cylinder 50 has a plurality of detection recesses 52a formed in an outer circumferential portion. A rotational position around the central axis O of the rotary cylinder 50 is detected by detecting combinations of the three limit switches 59a to 59c with the detection recesses 52a.

More specifically, the three limit switches 59a to 59c respectively have switch terminals 59A to 59C that contact with the outer circumferential surface of the rotational position detecting portion 52. On the outer circumferential surface of the rotational position detecting portion 52 against which the switch terminals 59A to 59C contact, combinations of one to three detection recesses 52a into which the switch terminals 59A to 59C are engageably inserted are formed.

That is, the rotational position detecting switch 59 detects a rotational position around the central axis O of the rotary cylinder 50 by binary combinations obtained by ON/OFF signals when switch terminals 59A to 59C of the three limit switches 59a to 59c are engageably inserted into the detection recesses 52a. The rotational positions of the rotary cylinder 50 are set to three rotating positions that are described later. In this connection, a maximum of seven rotation stop positions can be set with respect to the rotational positions of the rotary cylinder 50 by the three limit switches 59a to 59c and the plurality of detection recesses 52a.

A detection portion that detects a movement stopping position of the nozzle unit 70 provided in the rotary cylinder 50 is composed by the three limit switches 59a to 59c constituting the rotational position detecting switch 59 and the rotational position detecting portion 52 in which the detection recesses 52a are formed.

Further, the rotational position detecting switch 59 outputs a detection signal to the control portion 46 that is the controller shown in Fig. 2. Based on this detection signal, the control portion 46 stops the driving of the motor 57 to control the rotation around the central axis O of the rotary cylinder 50.

According to the present embodiment, a configuration has been described in which a rotating position around the central axis O of the rotary cylinder 50 is detected by detecting combinations of one to three detection recesses 52a on the outer circumferential surface of the rotational position detecting portion 52 using the three limit switches 59a to 59c. However, the present embodiment is not limited thereto. For example, a configuration may be adopted that detects a rotating position around the central axis O of the rotary cylinder 50 using various kinds of detection devices such as an optical sensor, or a configuration may be adopted that performs control to detect a rotating position around the central axis O of the rotary cylinder 50 using the number of rotations of a motor 57.

As shown in Fig. 7, in the conduit connection unit 5 that is configured and assembled as described above, the distal end frame 61 of the frame body 60 is mounted and fixed at a predetermined position in a wall portion of the washing bath 4 so that the front face of the nozzle housing 51 of the rotary cylinder 50 is exposed. In order that the fluid inside the washing bath 4 does not flow inside the apparatus, an airtightness maintaining member such as a gasket may be provided between the conduit connection unit 5 and the wall portion of the washing bath 4. Further, at an outer circumferential portion of the nozzle housing 51 is provided an O-ring 51b that is slidably placed in intimate contact with the inner circumferential face of the distal end frame 61 of the frame body 60 to retain water tightness.

The position at which the conduit connection unit 5 is installed to the washing bath 4 is the position at which the front portion of the nozzle housing 51 faces the channel connector portion 104 (see Fig. 9 and Fig. 10) as a conduit connector portion comprising an opening of the endoscope channel in a state in which the operation portion 103 of the endoscope 101 is positioned by the pins 4a of the washing bath 4.

The resistor 75a provided at the proximal end of the second nozzle pipe 75 of the conduit connection unit 5 has substantially the same outer diameter as the nozzle insertion hole portion 51a formed in the nozzle housing 51 of the rotary cylinder 50. More specifically, the second nozzle pipe 75 is configured to be slidable in a state in which the airtightness of the nozzle insertion hole portion 51a is maintained by the O-ring 75b provided on the resistor 75a and the outer circumferential surface thereof.

The resistor 75a also has, on the inside thereof, a recess 75c in which is formed a conical tapered face from the proximal end face to the distal end. The recess 75c communicates with a conduit of the second nozzle pipe 75.

The conduit of the second nozzle pipe 75 communicates with a conduit of the first nozzle pipe 74 and the gasket 76 of the conduit connector 71. In this connection, a conduit diameter φa of the second nozzle pipe 75 is set smaller than a conduit diameter φb of the tube connector pipe 56 (φa < φb).

Further, the nozzle insertion hole portion 5 1 a formed in the nozzle housing 51 is formed in a linear shape as far as midway along the inside of the rotational position detecting portion 52, and thereafter bends in a crank shape towards the center to constitute a fluid supply channel that opens at the rotation supporting portion 53.

In the rotation supporting portion 53 of the rotary cylinder 50, a circumferential groove is formed in the outer circumferential portion, and an O-ring 53a is provided in the circumferential groove. The O-ring 53a contacts with the inner circumferential face of the tube connection portion 63 and maintains airtightness between the tube connection portion 63 and the rotation supporting portion 53 that rotates around the central axis O.

Further, the nozzle housing 51 of the distal end portion is rotatingly held by the distal end frame 61, and the rotation supporting portion 53 of the proximal end portion is rotatingly held by the tube connection portion 63, and thus the rotary cylinder 50 is housed in the frame body 60 in a condition in which the rotary cylinder 50 can rotate around the central axis O.

During control in which the rotary cylinder 50 of the present embodiment is rotatingly driven around the central axis O, as shown in Fig. 8, the conduit connector 71 of the nozzle unit 70 housed in the nozzle housing 51 is, for example, set so as to be stopped at the three positions A to C. That is, since the nozzle unit 70 is set at a decentered position with respect to the central axis O of the rotary cylinder 50, the conduit connector 71 is controlled so as to rotatingly move along a circular orbit around the central axis O of the rotary cylinder 50 and stop at the aforementioned three positions A to C.

In the present embodiment, position A at which the conduit connector 71 of the nozzle unit 70 stops movement is a position at which the conduit connector 71 has moved to the uppermost part of the rotary cylinder 50, position B is a position at which the conduit connector 71 has moved to the lowermost part of the rotary cylinder 50, and position C is at an angle of 90° with respect to position A and is a position at which the conduit connector 71 has rotatingly moved around the central axis O of the nozzle housing 51 of the rotary cylinder 50 to the left side as viewed facing the page surface. That is, the positions A to C are positions at which the conduit connector 71 rotatingly moves by the amount of a 90° angle to each position, respectively, around the central axis O of the nozzle housing 51 of the rotary cylinder 50.

Among the three positions A to C, the two positions A and B at which movement of the conduit connector 71 is stopped correspond to an endoscope 101 provided with two channel connector portions 104 (see Fig. 9) as described later that are arranged in the operation portion 103 of the endoscope 101 as shown in Fig. 1. The two positions A and B are positions at which the respective channel connector portion 104 and the conduit connector 71 are positioned on the same axis and connect to each other.

Further, the position C at which movement of the conduit connector 71 is stopped corresponds to an endoscope 101 provided with one channel connector portion 104 (see Fig. 10) arranged in the operation portion 103 of the endoscope 101, and is a position at which the channel connector portion 104 and the conduit connector 71 are positioned on the same axis and connect to each other.

The endoscope washing and disinfecting apparatus 1 of the present embodiment configured as described above washes and disinfects the endoscope 101 that is mounted in the washing bath 4 according to a predetermined washing and disinfecting process that is programmed. Since the washing and disinfecting process executed by the endoscope washing and disinfecting apparatus 1 is performed in the same manner as in a conventional apparatus, a detailed description thereof is omitted.

Further, when the endoscope 101 to be washed is placed in the washing bath 4, the endoscope washing and disinfecting apparatus 1 reads an RFID or the like on which model information (endoscope ID) that is specific information of the endoscope 101 is stored, or the model information (endoscope ID) of the endoscope 101 is manually input by the user. Based on the model information of the endoscope 101, the endoscope washing and disinfecting apparatus 1 recognizes the number of channel connector portions 104 (one or two according to the present embodiment) provided in the operation portion 103, and control to drive the conduit connection unit 5 is executed by the control portion 46.

Hereafter, the actions of the conduit connection unit 5 when fluid is supplied into a channel of the endoscope 101 during the process to wash and disinfect the endoscope 101 using the endoscope washing and disinfecting apparatus 1 are described referring to Fig. 7 to Fig. 10. In this case, the fluid is washing water, disinfecting water, rinsing water, air, or alcohol.

First, a case in which the number of channel connector portions 104 provided in the operation portion 103 that is determined based on the model information (endoscope ID) of the endoscope 101 as read by the endoscope washing and disinfecting apparatus 1 or input by the user is a plurality is described. More specifically, in the following description the number of channel connector portions 104 is two.

In this connection, in a state in which fluid is not being supplied from the inside of the apparatus main body 2 via the automatic brush mechanism 80 and the fluid supply tube 83, in the conduit connection unit 5, as shown in Fig. 7, the flange 74b of the first nozzle pipe 74 of the nozzle unit 70 receives an urging force of the spring 73, and the first nozzle pipe 74 and second nozzle pipe 75 inside the nozzle housing 51 are urged in the proximal end direction, i.e. towards the rear. At this time, the conduit connector 71 that is connected to the first nozzle pipe 74 is in a state in which the conduit connector 71 is housed inside the recess 72a formed in the base 72.

In each washing and disinfecting step to wash and disinfect the endoscope 101, the endoscope washing and disinfecting apparatus 1 moves the conduit connector 71 of the nozzle unit 70 to a position on the same axis (position A in Fig. 8) as one of the channel connector portions 104 of the endoscope 101, as shown in Fig. 9. That is, by driving and stopping the motor 57 of the conduit connection unit 5 by means of control of the control portion 46, the endoscope washing and disinfecting apparatus 1 stops the rotating position of the rotary cylinder 50 at a position at which one of the channel connector portions 104 and the conduit connector 71 are aligned, based on detection signals of the rotational position detecting switch 59.

In this state, when fluid is supplied via the automatic brush mechanism 80 and the fluid supply tube 83 (see Fig. 7) from inside the apparatus main body 2, in the conduit connection unit 5, the resistor 75a of the second nozzle pipe 75 receives the fluid pressure and the first nozzle pipe 74 and the second nozzle pipe 75 move forward in resistance to the urging force of the spring 73. Thus, in the nozzle unit 70, the first nozzle pipe 74 is pushed out to the front so as to lead out from the recess 72a formed in the base 72 along with the conduit connector 71.

More specifically, when fluid from the fluid supply tube 83 is fed to the conduit connection unit 5, a pressure is applied to the resistor 75a of the second nozzle pipe 75. The fluid flows to a recess 75c formed in the proximal end face of the resistor 75a. Thereupon, as shown in Fig. 7, since the conduit diameter φa of the second nozzle pipe 75 is smaller than the conduit diameter φb of the nozzle insertion hole portion 51a of the nozzle housing 51, when fluid flows into the conduit of the second nozzle pipe 75, the fluid that passes through applies a force to the resistor 75a that pushes the resistor 75a forward.

In this connection, since the pressure applied by the fluid to the resistor 75a must be greater than an urging force of the spring 73, the fluid supply pressure of each pump and the compressor and the urging force of the spring 73 are set to a prescribed pressure or force.

The conduit connector 71 that moves in response to movement of the first nozzle pipe 74 and the second nozzle pipe 75 that are pushed outward upon receiving the pressure of the fluid is led out from inside the recess 72a formed in the base 72 towards the operation portion 103 of the endoscope 101. The conduit connector 71 of the nozzle unit 70 then enters a state of contact against one of the channel connector portions 104 of the operation portion 103 and is connected by the fluid pressure.

At this time, the gasket 76 provided in the conduit connector 71 enters a state of close contact with the opening of the channel connector portion 104. Therefore, fluid flowing inside the first nozzle pipe 74 and the second nozzle pipe 75 is supplied inside the channel provided in the endoscope 101 through one of the channel connector portions 104. Further, when the conduit connector 71 advances and connects with the channel connector portion 104, the tapered surface 71a formed in the front of the conduit connector 71 serves as a guide way so that the opening of the channel connector portion 104 is guided to the center of the conduit connector 71. Thus, even if positioning of the operation portion 103 of the endoscope 101 in the washing bath 4 at a predetermined position defined by the pins 4a is out of alignment to a certain extent, connection of the conduit connector 71 and the channel connector portion 104 is reliably performed.

At this time, the endoscope washing and disinfecting apparatus 1 drives the automatic brush mechanism 80 so that the cleaning brush 81 is introduced inside one of the channels of the endoscope 101 via the channel connector portion 104. The cleaning brush 81 is moved forward and backward for a predetermined time so that brushing and cleaning is carried out within one of the channels by the brush portion 82.

After carrying out brushing and cleaning within one of the channels for a predetermined time, the endoscope washing and disinfecting apparatus 1 houses the cleaning brush 81 inside the automatic brush mechanism 80 and temporarily stops supply of fluid to the channel of the endoscope 101. Thereupon, pressure produced by fluid to the resistor 75a stops, and by receiving the urging force of the spring 73 that contacts against the flange 74b of the first nozzle pipe 74, the first nozzle pipe 74 and the second nozzle pipe 75 are urged towards the proximal end direction, i.e. rearward, and housed inside the nozzle housing 51 of the rotary cylinder 50. At this time, since the conduit connector 71 connected to the first nozzle pipe 74 moves in response to movement of the first nozzle pipe 74 to the proximal end side so as to be housed inside the recess 72a formed in the base 72, the conduit connector 71 releases the connection with the channel connector portion 104.

Next, as shown in Fig. 9, the endoscope washing and disinfecting apparatus 1 moves the conduit connector 71 of the nozzle unit 70 to a position (position B in Fig. 8) on the same axis as the other channel connector portion 104 of the endoscope 101. That is, by driving and stopping the motor 57 of the conduit connection unit 5 by control of the control portion 46, the endoscope washing and disinfecting apparatus 1 stops the rotating position of the rotary cylinder 50 at a position at which the other channel connector portion 104 and the conduit connector 71 are aligned, based on detection signals of the rotational position detecting switch 59.

The endoscope washing and disinfecting apparatus 1 then controls the supply of fluid to the conduit connection unit 5 to cause the nozzle unit 70 to advance, similarly to the case of the first channel connector portion 104 described above, so that the conduit connector 71 connects with the other channel connector portion 104. The endoscope washing and disinfecting apparatus 1 then carries out brushing and cleaning for a prescribed time inside the other channel of the endoscope 101 that opens at the other channel connector portion 104.

As described above, the endoscope washing and disinfecting apparatus 1 of the present embodiment can carry out brushing and cleaning as well as disinfecting and the like inside a plurality of channels, in this case two channels, provided in the endoscope 101 using a single automatic brush mechanism 80.

A case will now be briefly described in which the model information (endoscope ID) of the endoscope 101 that is read by the endoscope washing and disinfecting apparatus 1 or input by the user indicates that the number of channel connector portions 104 provided in the operation portion 103 is one as shown in Fig. 10.

As shown in Fig. 10, in the endoscope washing and disinfecting apparatus 1, for each cleaning and disinfecting step that cleans and disinfects the endoscope 101, the conduit connector 71 of the nozzle unit 70 is moved to a position (position C in Fig. 8) on the same axis as the channel connector portion 104 of the endoscope 101. That is, by driving and stopping the motor 57 of the conduit connection unit 5 by means of control of the control portion 46, the endoscope washing and disinfecting apparatus 1 stops the rotating position of the rotary cylinder 50 at a position at which the channel connector portion 104 and the conduit connector 71 are aligned, based on detection signals of the rotational position detecting switch 59.

The endoscope washing and disinfecting apparatus 1 then controls the supply of fluid to the conduit connection unit 5 to cause the conduit connector 71 of the nozzle unit 70 to advance, as described above, so that the conduit connector 71 connects with the channel connector portion 104. The endoscope washing and disinfecting apparatus 1 then carries out brushing and cleaning for a prescribed time inside the channel of the endoscope 101 that opens at the channel connector portion 104. Thus, the endoscope washing and disinfecting apparatus 1 of the present embodiment can carry out brushing and cleaning as well as disinfecting and the like inside, in this case, a single channel that is provided in the endoscope 101.

As described above, when the endoscope washing and disinfecting apparatus 1 of the present embodiment carries out cleaning and disinfecting and the like inside a channel of the endoscope 101, by providing the conduit connection unit 5 with which the nozzle unit 70 that supplies a fluid into the channel can be attached and detached automatically, it is possible to adopt a configuration that decreases the amount of complicated operations for the user. Further, by means of the conduit connection unit 5, the endoscope washing and disinfecting apparatus 1 can move the nozzle unit 70 forward to a single or a plurality of channel connector portions 104 to correspond with endoscopes 101 of models in which the number of channels provided differ, and can selectively connect the nozzle unit 70 thereto.

As described in the foregoing, using the conduit connection unit 5, the endoscope washing and disinfecting apparatus 1 of the present embodiment can, in correspondence to each model of the endoscope 101, selectively and automatically attach and detach the nozzle unit 70 that supplies cleaning fluid, disinfecting fluid and the like to channels provided in the endoscope 101 with respect to each model, and thereby carry out washing and disinfecting together with brushing and cleaning inside a plurality of channels or a single channel.

Further, with the endoscope washing and disinfecting apparatus 1, since it is possible to selectively connect to a plurality of channels using the conduit connection unit 5, it is sufficient to provide only one automatic brush mechanism 80 to carry out brushing and cleaning inside the channels. That is, since it is not necessary to provided a plurality of automatic brush mechanisms 80 in the endoscope washing and disinfecting apparatus 1, not only is it sufficient to provide space for only a single automatic brush mechanism 80, but a configuration that is complicated due to providing multiple automatic brush mechanisms 80 is avoided and there is also an effect that costs can be reduced.

In this connection, although according to the present embodiment an example was illustrated in which the conduit connector 71 of the nozzle unit 70 rotatingly moves around the central axis O of the rotary cylinder 50 and is stopped at three positions A to C, as described above, a maximum of seven rotation stop positions can be set by combining the three limit switches 59a to 59c and the plurality of detection recesses 52a. More specifically, it is sufficient to appropriately set positions to which the conduit connector 71 of the nozzle unit 70 rotatingly moves and stops to the positions of the respective channel connector portion 104 according to the respective models of the endoscope 101.

### (Second Embodiment)

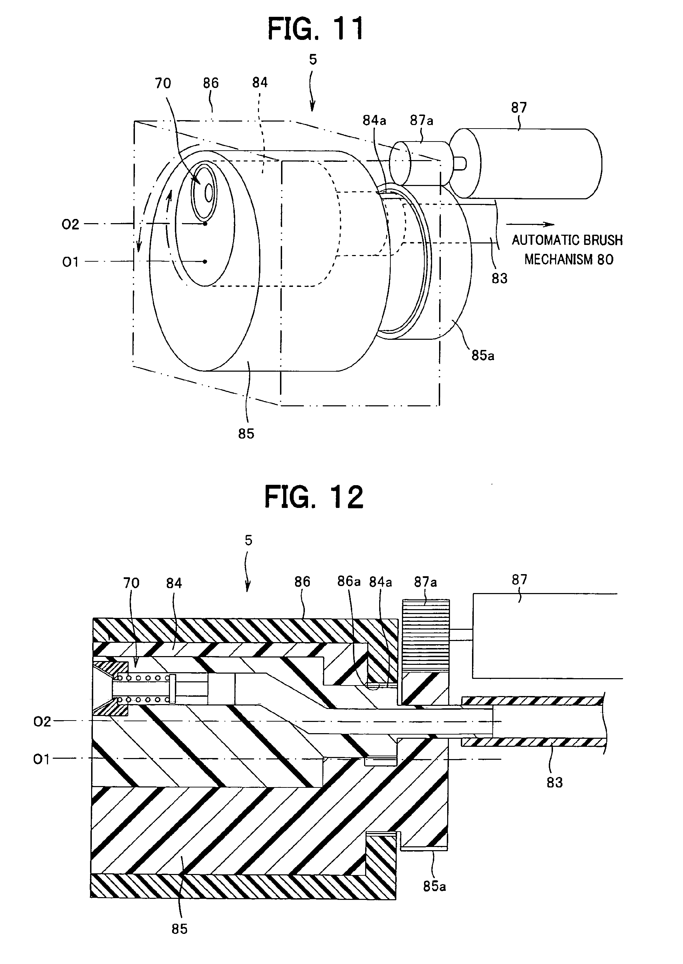

Next, a second embodiment of the present invention is described based on Fig. 11 to Fig. 20. In the following description, since only the configuration of the conduit connection unit 5 of the endoscope washing and disinfecting apparatus 1 of the present embodiment differs from the configuration described in the first embodiment, the same symbols as in the first embodiment are assigned to the same components, and a detailed description of those components is omitted.

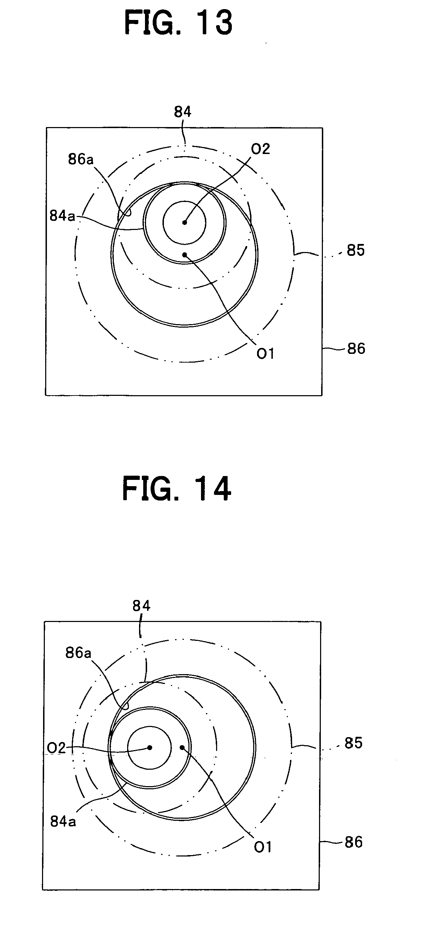

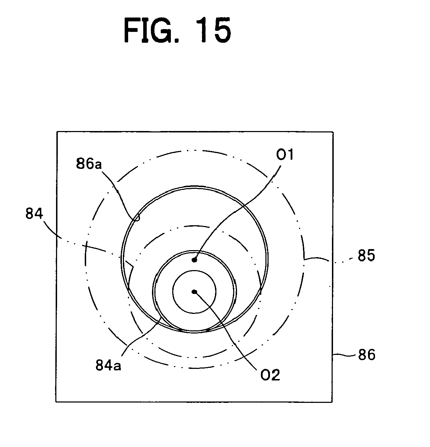

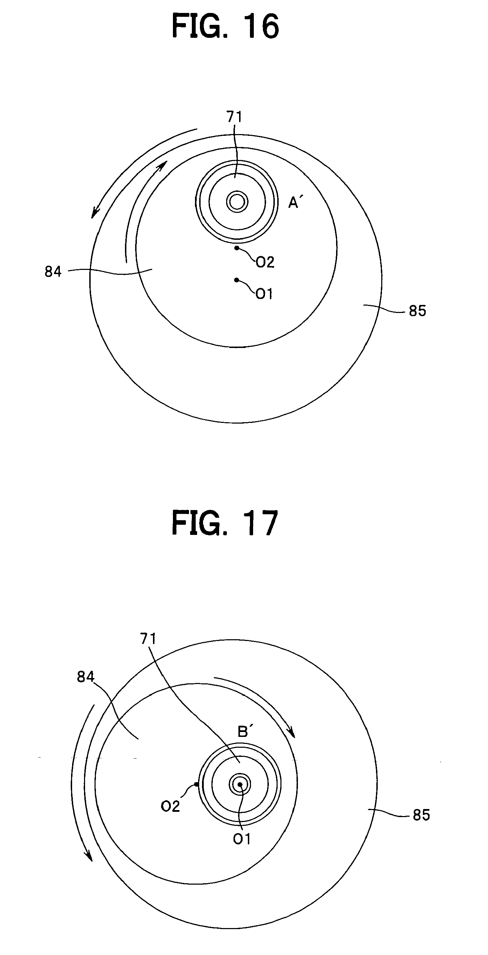

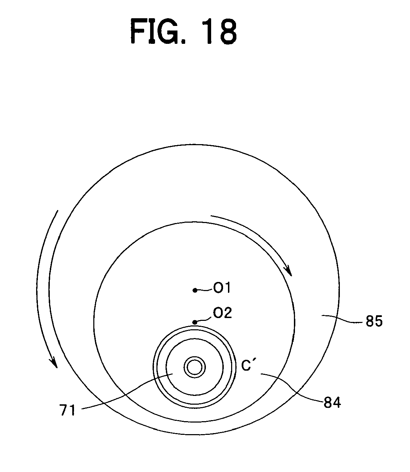

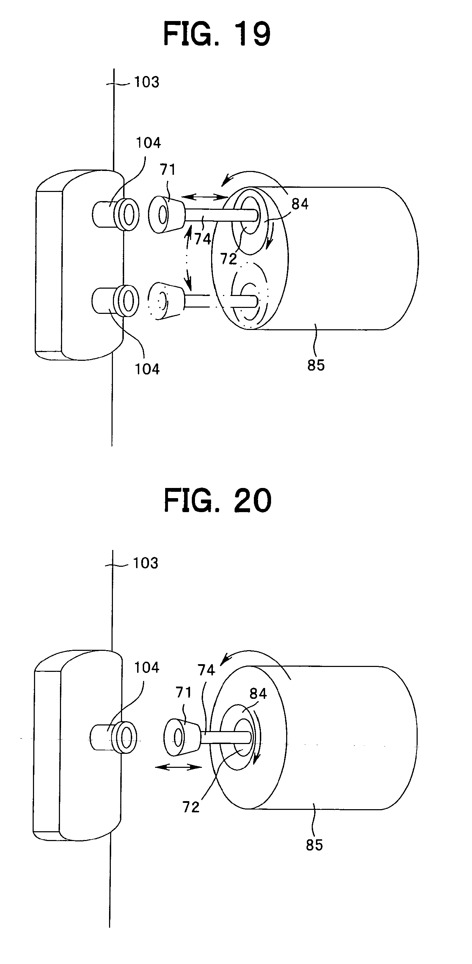

Fig. 11 to Fig. 20 relate to the second embodiment of the present invention. Fig. 11 is an oblique perspective view showing the configuration of a conduit connection unit. Fig. 12 is a cross-sectional view of the conduit connection unit. Fig. 13 is a view for describing a state in which, in response to rotation of an outer rotary body, an inner rotation side gear of an inner rotary body meshes with a frame body side gear of a frame body, and as a result the inner rotary body rotates around the central axis. Fig. 14 is a view for describing a state in which the outer rotary body makes a positive rotation by 90° from the state shown in Fig. 13, and the inner rotary body counter-rotates by 90° around the central axis. Fig. 15 is a view for describing a state in which the outer rotary body makes a positive rotation by 90° from the state shown in Fig. 14, and the inner rotary body counter-rotates by 90° around the central axis. Fig. 16 is a view for describing a state in which, by rotation of the inner rotary body around the central axis, a conduit connector of a nozzle unit of the inner rotary body moves. Fig. 17 is a view for describing a position to which the conduit connector of the nozzle unit moves upon the outer rotary body rotating by 90° from the state shown in Fig. 16. Fig. 18 is a view for describing a position to which the conduit connector of the nozzle unit moves upon the outer rotary body rotating by 90° from the state shown in Fig. 17. Fig. 19 is a view for describing actions of a conduit connection unit when an endoscope has two channel connector portions. Fig. 20 is a view for describing actions of the conduit connection unit when an endoscope has one channel connector portion.

As shown in Fig. 11 and Fig. 12, the conduit connection unit 5 of the present embodiment chiefly includes a box-shaped frame body 86 that is fixed to the washing bath 4, an outer rotary body 85 that is rotatably retained inside the frame body 86, an inner rotary body 84 that is rotatably retained inside the outer rotary body 85 and in which the nozzle unit 70 is housed at a decentered position, and a motor 87 as a driving source that rotatingly drives the outer rotary body 85 around a central axis O1.

The outer rotary body 85 has a substantially cylindrical shape and has an outer rotary body side gear 85a that meshes with the motor gear 87a of the motor 87 on an outer circumferential portion at the proximal end thereof. That is, rotation of the outer rotary body 85 around the central axis O is performed by driving of the motor 87.

The inner rotary body 84 has a substantially cylindrical shape and is provided in an inserted manner in a rotatable condition at a position that is decentered with respect to the central axis O1 of the outer rotary body 85. In the inner rotary body 84, a connecting pipe 84b provided at the proximal end thereof is inserted through the outer rotary body 85, and the connecting pipe 84b is connected to a fluid supply tube 83 that is connected to the automatic brush mechanism 80. In the inner rotary body 84, an inner rotary body side gear 84a is formed at an outer circumferential portion at a position that passes through the proximal end face of the frame body 86. In a state in which the inner rotary body 84 is provided in an inserted manner in the outer rotary body 85, one portion of the inner rotary body side gear 84a is exposed from the outer circumferential portion of the outer rotary body 85.

In the frame body 86, at an inner circumferential face of a hole portion at the proximal end face through which the outer rotary body 85 and the inner rotary body 84 are inserted is formed a frame body side gear 86a that meshes with the exposed portion of the inner rotary body side gear 84a of the inner rotary body 84 as described above. Further, in the frame body 86, an unshown airtightness and watertightness maintaining member such as a gasket is provided such that the rotary bodies 84 and 85 are exposed in the wall surface of the washing bath 4, and is fixed in a state that maintains the airtightness and watertightness. In this connection, the frame body 86, the outer rotary body 85, and the inner rotary body 84 are provided with an unshown airtightness and watertightness maintaining member such as an O-ring, and the airtightness and watertightness maintaining members are installed in a state that maintains mutual airtightness and watertightness.

In the conduit connection unit 5 of the present embodiment that is configured as described above, the outer rotary body 85 is rotated around the central axis O1 by the motor 87. At this time, since the inner rotary body 84 is arranged at a position that is decentered from the central axis O1 of the outer rotary body 85, while moving along a circular orbit around the central axis O1 the inner rotary body 84 rotates around its own central axis 02 by meshing between the frame body side gear 86a of the frame body 86 and the inner rotary body side gear 84a.

More specifically, as shown in Fig. 13 to Fig. 15, in the conduit connection unit 5, when the outer rotary body 85 is rotated in a counterclockwise direction as viewed from the front around the central axis O1 by the motor 87, the inner rotary body 84 moves along a circular orbit around the central axis O1. At this time, by the inner rotary body side gear 84a meshing with the frame body side gear 86a, the inner rotary body 84 rotates in response thereto in a clockwise direction around the central axis 02. In other words, by the inner rotary body side gear 84a meshing with the frame body side gear 86a, the inner rotary body 84 rotates around the central axis 02 in the opposite direction to the rotational direction around the central axis O1 of the outer rotary body 85.

When the outer rotary body 85 rotates around the central axis O1, as shown in Fig. 16 to Fig. 18, in the nozzle unit 70 arranged in the inner rotary body 84, the conduit connector 71 that is disposed at the front portion of the inner rotary body 84 moves to position A' to C'. More specifically, the inner rotary body 84 rotates in the clockwise direction in response to rotation in the counterclockwise direction by the outer rotary body 85, and thus the position of the conduit connector 71 of the nozzle unit 70 moves up or down in a straight manner between the positions A' to C' in Fig. 16 to Fig. 18.

In this connection, in the conduit connection unit 5 of the present embodiment, the number of teeth of the outer rotary body side gear 85a of the outer rotary body 85 and the number of teeth with which the frame body side gear 86a of the frame body 86 and the inner rotary body side gear 84a respectively mesh, are arranged so that the amount of rotation of the outer rotary body 85 and the amount of rotation of the inner rotary body 84 are equal.

More specifically, when the outer rotary body 85 rotates 90° in the counterclockwise direction from a state in which the conduit connector 71 is at position A' as shown in Fig. 16, the inner rotary body 84 rotates 90° in the clockwise direction so that the conduit connector 71 moves to position B' as shown in Fig. 17. Further, when the outer rotary body 85 again rotates 90° in the counterclockwise direction from the state in which the conduit connector 71 is at position B' as shown in Fig. 17, the inner rotary body 84 rotates 90° in the clockwise direction so that the conduit connector 71 moves to position C' as shown in Fig. 18.

Although not shown in the figures, the conduit connection unit 5 is provided with three limit switches 59a to 59c similarly to the first embodiment, with which the rotational position of the outer rotary body 85 is detected to control driving and stopping of the motor 87. That is, the endoscope washing and disinfecting apparatus 1 is configured so that control is performed to drive and stop rotation of the outer rotary body 85 at, for example, the three positions A' to C' by means of the control portion 46.

That is, as shown in Fig. 19, with the conduit connection unit 5, when the endoscope 101 is a model that is equipped with two channel connector portions 104, the outer rotary body 85 is rotatingly controlled to move to two positions at which the conduit connector 71 of the nozzle unit 70 advances and connects thereto (for example, the above described position A' and C'), and in response to the rotation of the outer rotary body 85, the inner rotary body 84 rotates. Further, as shown in Fig. 20, with the conduit connection unit 5, when the endoscope 101 is a model that is equipped with one channel connector portion 104, the outer rotary body 85 is rotatingly controlled to move to a single position at which the conduit connector 71 of the nozzle unit 70 advances and connects thereto (for example, the above described position B'), and in response to the rotation of the outer rotary body 85, the inner rotary body 84 rotates.

Accordingly, in the endoscope washing and disinfecting apparatus 1 of the present embodiment, by configuring the conduit connection unit 5 as described above, the conduit connector 71 of the nozzle unit 70 can be set so as to move on the same axis as the channel connector portion(s) 104 of the endoscope 101 and connect thereto at, for example, the three movement positions A' to C'. In this connection, similarly to the first embodiment, in the endoscope washing and disinfecting apparatus 1, control performed by the control portion 46 can be set to drive and stop rotation of the outer rotary body 85 so as to position the outer rotary body 85 at a maximum of seven positions.

That is, although according to the first embodiment a configuration is adopted in which the conduit connector 71 of the nozzle unit 70 moves in a circular orbit around the central axis O of the rotary cylinder 50, with the conduit connection unit 5 of the present embodiment the inner rotary body 84 counter-rotates in response to a positive rotation by the outer rotary body 85 so that the conduit connector 71 of the nozzle unit 70 can be linearly moved between the positions A' to C'.

The remaining configuration and actions of the endoscope washing and disinfecting apparatus 1 of the present embodiment are the same as in the first embodiment, and hence a description thereof is omitted here.

Thus, the endoscope washing and disinfecting apparatus 1 of the present embodiment can achieve the same advantages as the first embodiment, and even in a case where the respective disposition positions of a single or a plurality of channel connector portions 104 provided in the operation portion 103 of the endoscope 101 in respective models thereof are provided on a straight line that connects the positions A' to C', it is possible to set the conduit connector 71 of the nozzle unit 70 so as to move to a connecting position on the same axis as each channel connector portion 104 for each model of the endoscope 101. (Third Embodiment)

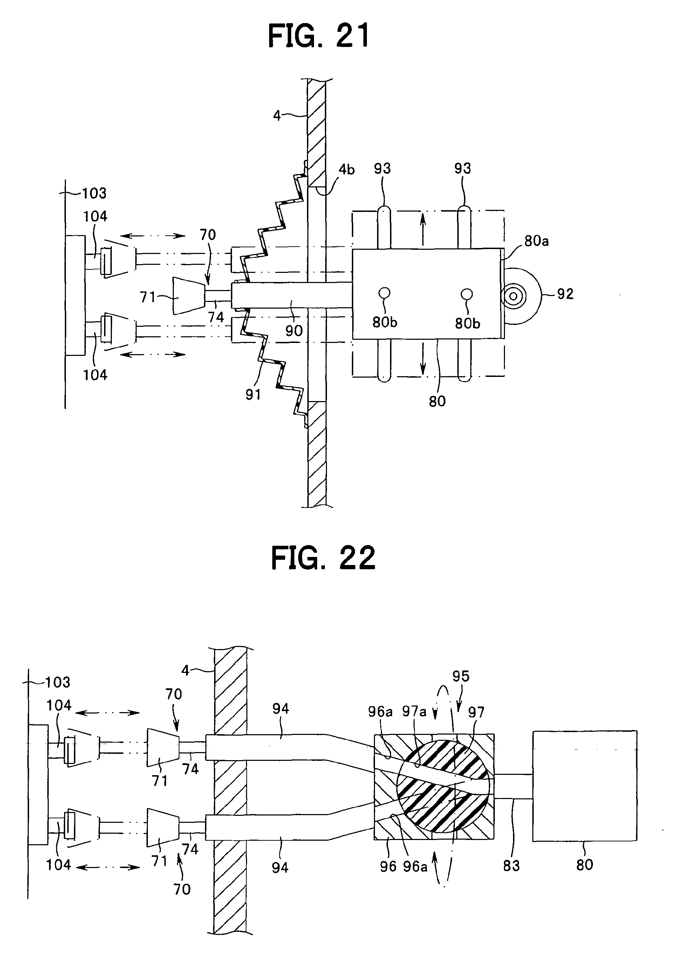

Next, a third embodiment of the present invention is described referring to Fig. 21. Fig. 21 is a partial cross-sectional view that illustrates an automatic brush mechanism that is moved in two directions by a motor, and a configuration in which a nozzle unit is provided in the automatic brush mechanism, according to the third embodiment of the present invention.

The conduit connection unit 5 of the endoscope washing and disinfecting apparatus 1 of the present embodiment is a different shape to that of the above described embodiments, and is configured to attach and detach the nozzle unit 70 to a plurality of the channel connector portions 104 of the endoscope 101. In the following description, the same symbols as in each of the above embodiments are assigned to the same components, and a detailed description of those components is omitted.

As shown in Fig. 21, in the endoscope washing and disinfecting apparatus 1, the nozzle unit 70 that is moveably inserted into a hole portion 4b in a wall portion of the washing bath 4 is arranged so as to be capable of moving in two directions (upward and downward in Fig. 21) within the area of the hole portion 4b. Further, the nozzle unit 70 is housed in the nozzle housing pipe 90 and the nozzle housing pipe 90 is connected to the automatic brush mechanism 80.

The distal end portion of the nozzle housing pipe 90 is arranged so as to pass through an accordion-shaped waterproof seal member 91 in a state in which the watertightness thereof is maintained. The waterproof seal member 91 is fixed so as to maintain the watertightness of the hole portion 4b of the washing bath 4.

The automatic brush mechanism 80 includes a gear groove 80a as a rack at the rear end face thereof. The gear groove 80a is meshed with a motor gear 92a of a motor 92 as a pinion. The automatic brush mechanism 80 is engageably inserted into two rails 93 provided inside the apparatus main body 2, and includes two pin members 80b that are guided in a straight line. That is, the automatic brush mechanism 80 is moved in two directions, upward and downward, in Fig. 21 along the two rails 93 by driving of the motor 92.

In response to this movement, the nozzle housing pipe 90 connected to the automatic brush mechanism 80 also moves in two directions, upward and downward, in Fig. 21. The automatic brush mechanism 80 is configured so that driving of the motor 92 is controlled by the control portion 46 such that the nozzle unit 70 provided in the nozzle housing pipe 90 moves to two positions on the same axis, respectively, as two channel connector portions 104 of the endoscope 101 in this case. Further, according to the present embodiment, the amount of movement in two directions of the automatic brush mechanism 80 is set by the number of rotations of the motor gear 92a of the motor 92 so that the nozzle unit 70 is moved onto the same axis, respectively, as the two channel connector portions 104.

More specifically, when the nozzle unit 70 has moved to and stopped on the same axis as one of the channel connector portions 104, by supplying fluid to the nozzle housing pipe 90 via the automatic brush mechanism 80, the conduit connector 71 receives the pressure of the fluid and is caused to advance to be connected to the channel connector portion 104 in question. Likewise, when the nozzle unit 70 has moved to and stopped on the same axis as the other channel connector portion 104, by supplying fluid to the nozzle housing pipe 90 via the automatic brush mechanism 80, the conduit connector 71 receives the pressure of the fluid and is caused to advance to be connected to the other channel connector portion 104.

The remaining configuration and actions of the endoscope washing and disinfecting apparatus 1 of the present embodiment are the same as in the first embodiment, and hence a description thereof is omitted here.

As described above, the endoscope washing and disinfecting apparatus 1 of the present embodiment is configured to control movement of the automatic brush mechanism 80 in two directions so that the nozzle unit 70 can connect to each channel connector portion 104. By adopting this configuration, the endoscope washing and disinfecting apparatus 1 can achieve the same advantages as each of the embodiments described above and, further, a mechanism that connects the nozzle unit 70 to each channel connector portion 104 of the endoscope 101 can be simplified.

### (Fourth Embodiment)

Next, a fourth embodiment of the present invention is described referring to Fig. 22. Fig. 22 is a partial cross-sectional view for describing a conduit switching mechanism portion that switches flow channels that is inserted into two nozzle units according to the fourth embodiment of the present invention.

In the present embodiment, the conduit connection unit 5 is a different shape to that of each of the above described embodiments, and is configured to individually and respectively attach and detach two nozzle units 70 to a plurality of channel connector portions 104 of the endoscope 101. In the following description, the same symbols as in each of the above described embodiments are assigned to the same components, and a detailed description of those components is omitted.

As shown in Fig. 22, in the endoscope washing and disinfecting apparatus 1 of the present embodiment, two nozzle units 70 arranged so as to be movable forward and backward in nozzle housing pipes 94 are provided in a wall face of the washing bath 4 so as to be positioned on the same axis, respectively, as two channel connector portions 104 of the endoscope 101 provided in the washing bath 4. More specifically, the nozzle housing pipes 94 which respectively fitted over the two nozzle units 70 are fixed in a state in which watertightness is maintained to the wall surface of the washing bath 4 so as to extend to inside the apparatus main body 2.

These two nozzle housing pipes 94 are connected so as to communicate with a conduit switching mechanism 95 inside the apparatus main body 2. The conduit switching mechanism 95 is connected to the automatic brush mechanism 80 via a fluid supply tube 83.

The conduit switching mechanism 95 has an outer frame 96 and a spherical ball valve 97 that is rotatably provided inside the outer frame 96. The outer frame 96 includes two flow channels 96a that respectively communicate with the two nozzle housing pipes 94 to be connected.

In the conduit switching mechanism 95, by control performed by the control portion 46, switching can be selectively performed to rotate the ball valve 97 using an unshown driving apparatus so that one of the two flow channels 96a of the outer frame 96 and an inner channel 97a formed inside the ball valve 97 communicate with the automatic brush mechanism 80 via the fluid supply tube 83.

More specifically, in the conduit switching mechanism 95, in order that one of the nozzle housing pipes 94 and the automatic brush mechanism 80 communicate, the ball valve 97 is rotatingly controlled to make the inner channel 97a and one of the flow channels 96a communicate. Then, by supplying fluid to one of the nozzle housing pipes 94 via the automatic brush mechanism 80, the nozzle unit 70 receives the pressure of the fluid and is caused to advance to be connected to the channel connector portion 104 in question. Further, when the ball valve 97 of the conduit switching mechanism 95 is switched and fluid is supplied through the automatic brush mechanism 80 to the other nozzle housing pipe 94, the nozzle unit 70 receives the pressure of the fluid and is caused to advance to be connected to the other channel connector portion 104.

The remaining configuration and actions of the endoscope washing and disinfecting apparatus 1 of the present embodiment are the same as in the first embodiment, and hence a description thereof is omitted here.

By adopting the above described configuration, the endoscope washing and disinfecting apparatus 1 of the present embodiment provides a simple structure that enables connection between two nozzle units 70 and each channel connector portion 104.

### (Fifth Embodiment)

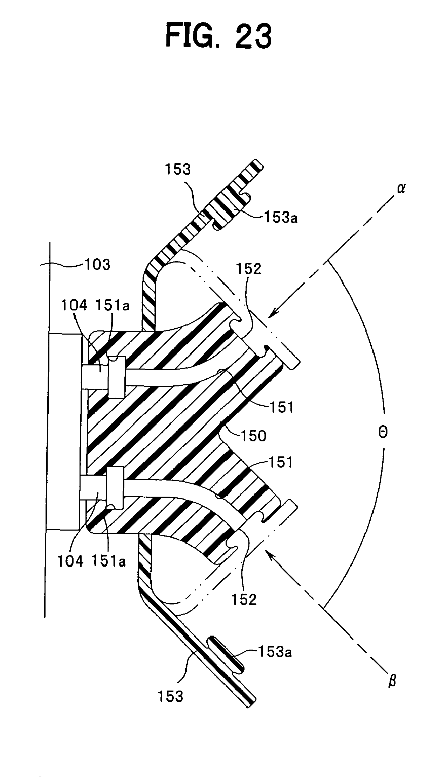

Next, a fifth embodiment of the present invention is described referring to Fig. 23. Fig. 23 is a cross-sectional view that illustrates a forceps plug that is detachably mounted to two channel connector portions arranged in an operation portion of an endoscope according to the fifth embodiment of the present invention.

In a case where the endoscope 101 is equipped with two channel connector portions 104 that are provided in the operation portion 103, a configuration in which the nozzle unit 70 moves forward and backward at a position on the same axis as the two channel connector portions is described for the endoscope washing and disinfecting apparatus 1 according to each of the above embodiments. That is, it is necessary for the two channel connector portions 104 of the endoscope 101 to be parallelly disposed in the operation portion 103.

However, when a physician as a user of the endoscope 101 inserts or extracts a medical instrument such as a treatment instrument into two parallel channels of the endoscope 101, because the respective channel connector portions 104 that are the insertion openings of the two channels are parallel and adjacent to each other, a problem arises that the insertability of the medical instrument into each channel connector portion 104 is poor, and the workability declines.

Therefore, according to the present embodiment, a forceps plug 150 shown in Fig. 23 that can be detachably mounted to the channel connector portion 104 is described. The forceps plug 150 can be attached to the endoscope 101 at a time of use, and can be removed from the endoscope 101 when washing and disinfecting are performed by the endoscope washing and disinfecting apparatus 1 according to the respective embodiments described above.

As shown in Fig. 23, the forceps plug 150 is made of a synthetic resin that has elasticity, such as elastomer. Two inserting sections 151 are formed in the forceps plug 150 in order to allow a medical instrument such as a treatment instrument to pass therethrough. In the forceps plug are formed two recesses 152 at which one end of each inserting section 151 opens and each of which constitutes an insertion port. Two sealing plug bodies 153 that can open and close and that each includes an engaging protrusion 153a are engageably inserted into the recesses 152 to hermetically seal the inserting sections 151.

On the other end sides of the inserting sections 151 of the forceps plug 150 are formed engaging recesses 151 a into which the two channel connector portions 104 of the endoscope 101 are engageably fixed. That is, because the forceps plug 150 has elasticity, the engaging recesses 151a are fitted and detachably fixed to each of the two channel connector portions 104 of the endoscope 101. At this time, each inserting section 151 communicates with a channel of the endoscope 101 via the respective channel connector portion 104.

The two inserting sections 151 are formed so that the recesses 152 comprising the insertion ports are bent in directions away from each other at a predetermined angle θ, respectively. That is, the openings of the two recesses 152 serving as insertion ports of the forceps plug 150 are arranged so as to face directions having a predetermined angle θ.

The forceps plug 150 of the present embodiment that is configured as described above is attached to the endoscope 101 during operations. The user inserts and extracts a medical instrument such as a treatment instrument into and out from the two channels of the endoscope 101 through the forceps plug 150.

At this time, since a direction (for example, the direction indicated by the arrow α in the figure) for inserting and extracting a medical instrument into and out from one of the channels of the endoscope 101 and a direction (for example, the direction indicated by the arrow (β in the figure) for inserting and extracting a medical instrument into and out from the other channel of the endoscope 101 are separated by a predetermined angle θ by the forceps plug 150, the user can easily insert and extract the medical instrument into and out from each channel. Thus, the insertability of a medical instrument into each channel is improved by the forceps plug 150. Further, when a used endoscope 101 is washed and disinfected with the endoscope washing and disinfecting apparatus 1, the forceps plug 150 is removed.

As described above, the workability for a user when a medical instrument is inserted into or extracted out from a channel of the endoscope 101 during an operation is improved by the forceps plug 150 of the present embodiment. Further, by removing the forceps plug 150 from the endoscope 101, the user can wash and disinfect a used endoscope 101 with the endoscope washing and disinfecting apparatus 1 according to the above described embodiments.

### (Sixth Embodiment)

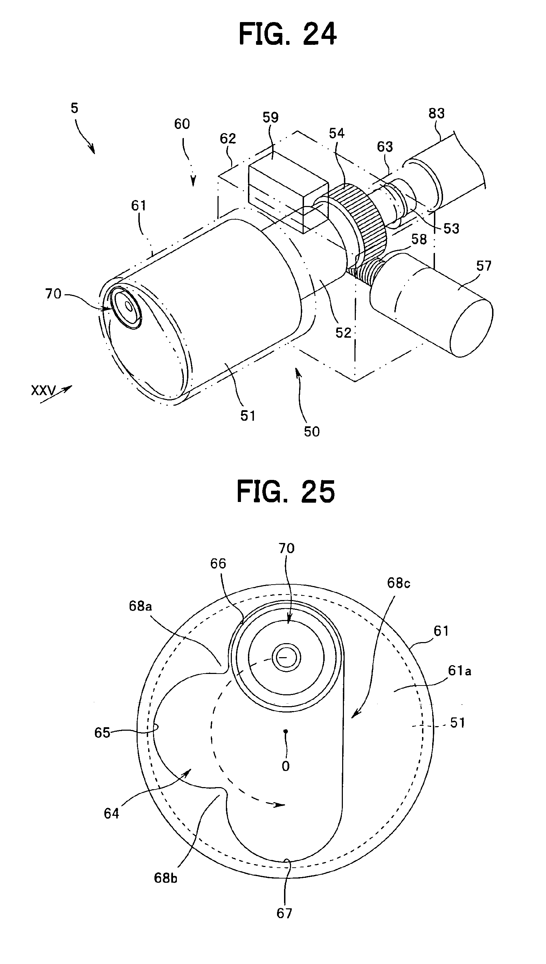

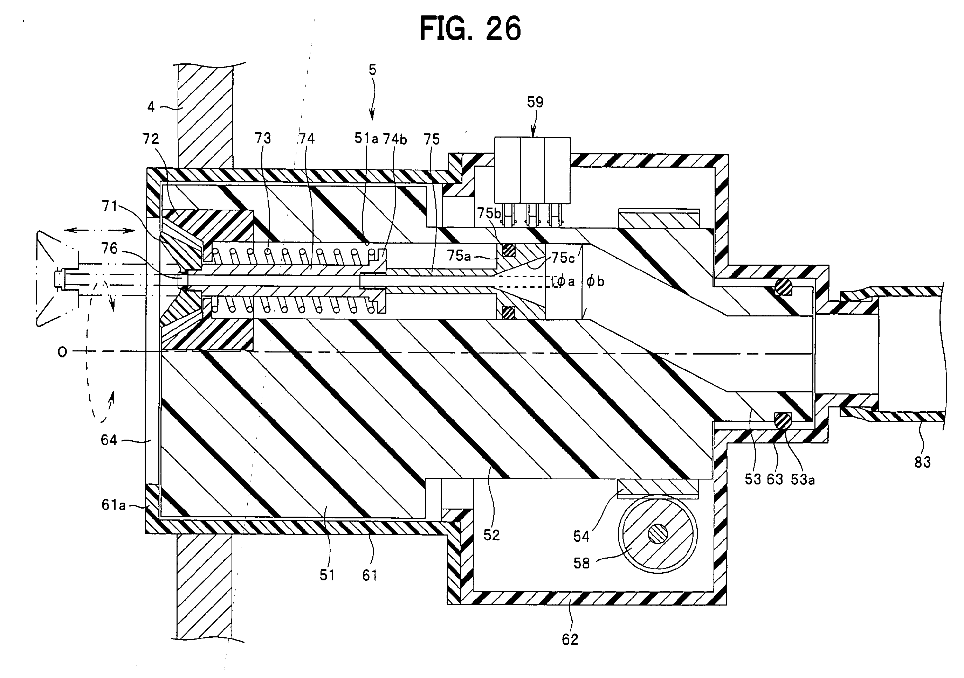

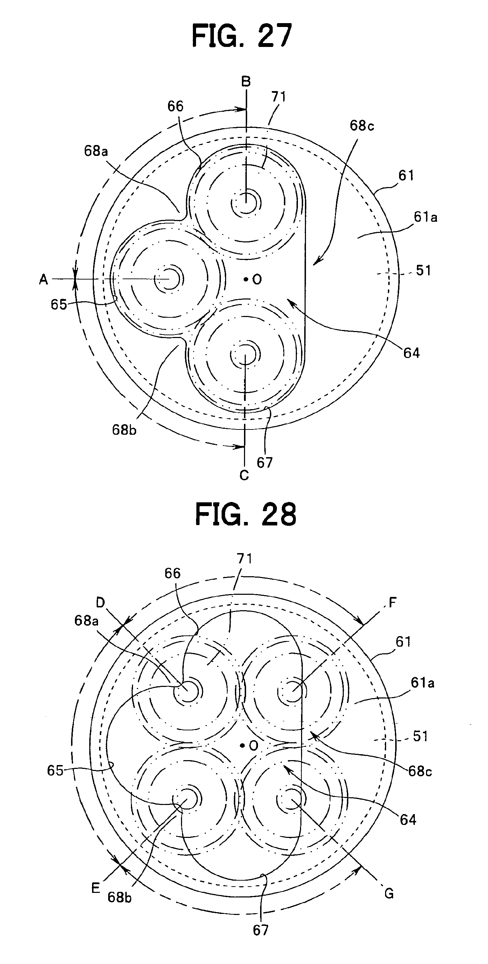

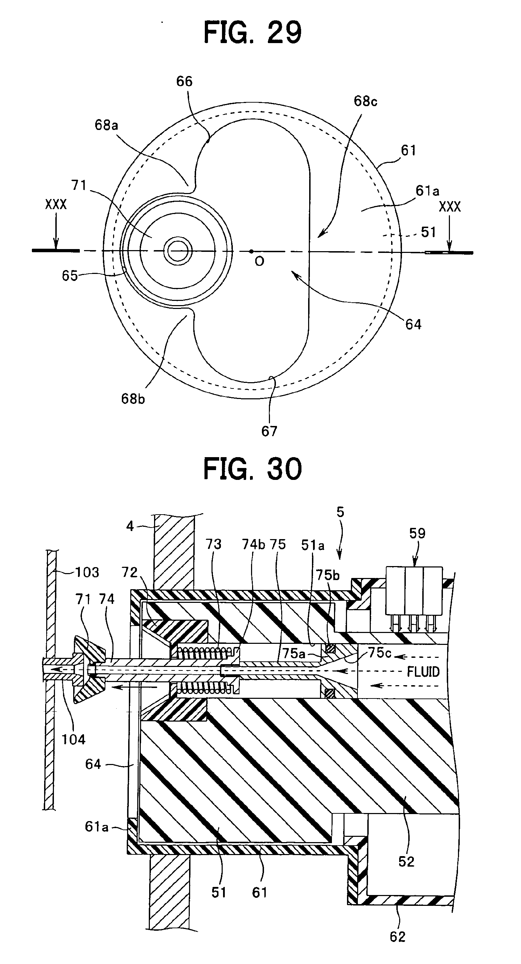

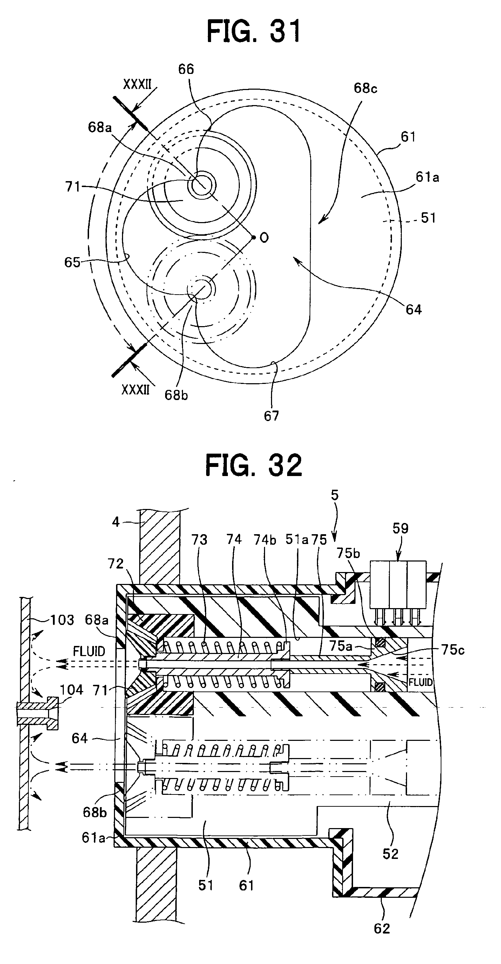

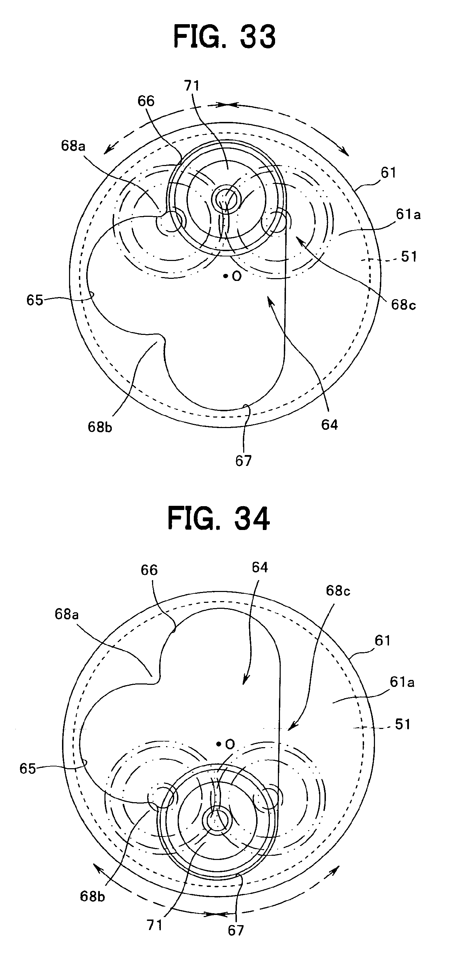

Next, a sixth embodiment of the present invention is described below based on Fig. 24 to Fig. 34. Fig. 24 to Fig. 34 relate to the sixth embodiment of the present invention. Fig. 24 is an oblique perspective view that illustrates the configuration of a conduit connection unit. Fig. 25 is a plan view of the conduit connection unit shown in Fig. 24 as viewed from the direction of the arrow XXV. Fig. 26 is a cross-sectional view of the conduit connection unit that is disposed in a washing bath. Fig. 27 is a view for describing a plurality of positions at which a nozzle unit is connected to a channel connector portion of an endoscope. Fig. 28 is a view for describing a plurality of positions at which a nozzle unit abuts against and is regulated by a nozzle abutment portion. Fig. 29 is a front view of the conduit connection unit for describing an action through which the nozzle unit is connected to the channel connector portion of an endoscope. Fig. 30 is a cross-sectional view of the conduit connection unit taken along the line XXX-XXX in Fig. 29. Fig. 31 is a front view of the conduit connection unit for describing an action at two positions at which the nozzle unit abuts against and is regulated by the nozzle abutment portion. Fig. 32 is a cross-sectional view of the conduit connection unit taken along the line XXXII-XXXII shown in Fig. 31. Fig. 33 is a front view of the conduit connection unit for describing, in correspondence to an endoscope including two channel connector portions, a state in which the nozzle unit is connected to one of the channel connector portions, and a state in which the nozzle unit abuts against and is regulated by the nozzle abutment portion. Fig. 34 is a front view of the conduit connection unit for describing, in correspondence to an endoscope including two channel connector portions, a state in which the nozzle unit is connected to the other channel connector portion, and a state in which the nozzle unit abuts against and is regulated by the nozzle abutment portion.

In the following description, the same symbols as used in each of the above embodiments are assigned to the same components, and a detailed description of those components is omitted.

First, based on Fig. 24 to Fig. 27, the configuration of the conduit connection unit 5 of the present embodiment that is provided inside the apparatus main body 2 such that the distal end portion is exposed in the washing bath 4 is described in detail.

As shown in Fig. 24, similarly to each of the above described embodiments, the conduit connection unit 5 of the present embodiment has a rotary cylinder 50 as a rotating member in which the aforementioned nozzle unit 70 (see Fig. 5) as a washing nozzle can move forward and rearward, and a frame body 60 in which the rotary cylinder 50 is housed in a rotatable condition.

In the frame body 60 of the present embodiment, the distal end frame 61 that rotatably supports and houses the nozzle housing 51 has a distal end face 61 a that is provided so as to cover one portion of the distal end face of the nozzle housing 51.

As shown in Fig. 25, the distal end face 61a of the distal end frame 61 has an opening 64 which is formed so that three circular holes are connected at positions that are staggered at intervals of an angle of 90° according to the present embodiment around the central axis O of the housed rotary cylinder 50. The three circular holes forming the opening 64 include, as viewed from the direction facing the surface of the sheet showing Fig. 25, a first nozzle outlet opening 65 that is positioned on the center left side, a second nozzle outlet opening 66 that is position on the upper side, and a third nozzle outlet opening 67 that is positioned underneath.

These first to third nozzle outlet openings 65 to 67 have such a size and a shape that the nozzle unit 70 can be led out from the nozzle housing 51 by the nozzle unit 70 that is stored in the rotary cylinder 50 rotatingly moving around the central axis O in alignment the respective positions.

Further, at the distal end face 61a of the distal end frame 61 are formed: a first nozzle abutment portion 68a that protrudes towards the center at a position between the first nozzle outlet opening 65 and the second nozzle outlet opening 66, a second nozzle abutment portion 68b that protrudes towards the center at a position between the first nozzle outlet opening 65 and the third nozzle outlet opening 67, and a third nozzle abutment portion 68c at an edge portion that connected the second nozzle outlet opening 66 and the third nozzle outlet opening 67. The first nozzle abutment portion 68a to third nozzle abutment portion 68c constitute a regulating portion that regulates frontward movement of the nozzle unit 70 that is led out from the rotary cylinder 50.

Further, the rotational position detecting switch 59 of the present embodiment detects a rotational position around the central axis O of the rotary cylinder 50 by binary combinations obtained by ON/OFF signals when switch terminals 59A to 59C of the three limit switches 59a to 59c are engageably inserted into detection recesses 52a. The rotational positions of the rotary cylinder 50 are set to seven rotating positions that are described later.

In the present embodiment, a detection portion that detects a movement position of the nozzle unit 70 provided in the rotary cylinder 50 is composed by the three limit switches 59a to 59c constituting the rotational position detecting switch 59 and the rotational position detecting portion 52 in which the detection recesses 52a are formed.

The conduit connection unit 5 that is configured and assembled as described above is mounted and fixed so that the distal end face 61 a of the distal end frame 61 of the frame body 60 is exposed at a prescribed position of a wall portion of the washing bath 4 as shown in Fig. 26. In this connection, an airtightness maintaining member such as a gasket may be provided between the conduit connection unit 5 and the wall portion of the washing bath 4 so that fluid inside the washing bath 4 does not flow into the apparatus.

The position at which the conduit connection unit 5 is mounted to the washing bath 4 is a position at which the distal end face 61 a of the distal end frame 61 faces the channel connector portion 104 (see Fig. 30 and Fig. 32) that is a conduit connector portion comprising an opening of an endoscope channel in a state in which the operation portion 103 of the endoscope 101 is positioned by the pins 4a of the washing bath 4.

In this connection, the resistor 75a provided at the proximal end of the second nozzle pipe 75 of the conduit connection unit 5 has substantially the same outer diameter as the nozzle insertion hole portion 51a formed in the nozzle housing 51 of the rotary cylinder 50. More specifically, the second nozzle pipe 75 is arranged so as to be slidable in a state in which airtightness is maintained with respect to the nozzle insertion hole portion 51a by the O-ring 75b provided in the resistor 75a and the outer circumferential surface of the second nozzle pipe 75 itself.

As described above, in the conduit connection unit 5 of the present embodiment, rotation of the rotary cylinder 50 is driven to and stopped at seven rotating positions that are detected by means of combinations of the three limit switch 59a to 59c constituting the rotational position detecting switch 59 with the plurality of detection recesses 52a of the rotational position detecting portion 52.

More specifically, during control in which the rotary cylinder 50 is rotatingly driven around the central axis O, the conduit connector 71 of the nozzle unit 70 housed in the nozzle housing 51 is stopped at a total of seven positions formed in the distal end face 61 a of the distal end frame 61. These seven positions include three positions A to C that match the first to third nozzle outlet openings 65 to 67 as shown in Fig. 27, and four positions D to G that match the first to third nozzle abutment portions 68a to 68c shown in Fig. 28. That is, since the nozzle unit 70 is set at a decentered position with respect to the central axis O of the rotary cylinder 50, the conduit connector 71 is controlled so as to rotatingly move around the central axis O of the rotary cylinder 50 and stop at the aforementioned seven positions A to G.

In the present embodiment, the position A to which the conduit connector 71 of the nozzle unit 70 moves to and stops at in the first nozzle outlet opening 65 corresponds to an endoscope 101 as shown in Fig. 1 that includes a single channel connector portion 104, described later, (see Fig. 30 and Fig. 32) provided in the operation portion 103 of the endoscope 101, and is the position at which the channel connector portion 104 and the conduit connector 71 are located on the same axis and are connected to each other.

Further, in the present embodiment, the positions B and C to which the conduit connector 71 of the nozzle unit 70 moves to and stops at in the second nozzle outlet opening 65 and the third nozzle outlet opening 67 correspond to an endoscope 101 as shown in Fig. 1 that includes two channel connector portions 104, described later, provided in the operation portion 103 of the endoscope 101, and are the positions at which the two channel connector portions 104 and the nozzle unit 70 are located on the same axis, respectively, and are connected to each other.

Moreover, the four positions D to G to which the conduit connector 71 of the nozzle unit 70 moves to and stops at are four positions that are respectively staggered at intervals of a prescribed angle of, according to the present embodiment, 45° with respect to the neighboring positions A to C around the central axis O of the rotary cylinder 50. At these four positions D to G, the nozzle unit 70 is in a state in which the nozzle unit 70 abuts against any of the first to third nozzle abutment portions 68a to 68c, and is not positioned on the same axis as the channel connector portion 104 of the endoscope 101.

That is, these four positions D to G are positions that are staggered by the distance that the conduit connector 71 of the nozzle unit 70 is moved by rotation of the rotary cylinder 50 from any of the three positions A to C at which the channel connector portion 104 of the endoscope 101 is on the same axis as the conduit connector 71 of the nozzle unit 70.

The endoscope washing and disinfecting apparatus 1 of the present embodiment configured as described above washes and disinfects the endoscope 101 that is mounted in the washing bath 4 according to a predetermined washing and disinfecting process that is programmed. The endoscope washing and disinfecting apparatus 1 reads an RFID or the like on which model information (endoscope ID) that is specific information of the endoscope 101 to be washed is stored, or the model information (endoscope ID) of the endoscope 101 is manually input by the user. Based on the model information of the endoscope 101, the endoscope washing and disinfecting apparatus 1 recognizes the number of channel connector portions 104 (in this case, one or two) provided in the operation portion 103, and control to drive the conduit connection unit 5 is executed by the control portion 46.

Hereunder, the actions of the conduit connection unit 5 when fluid is supplied into a channel of the endoscope 101 during a process to wash and disinfect the endoscope 101 using the endoscope washing and disinfecting apparatus 1 are described referring to Fig. 26 to Figs. 29 to 32. In this case, the fluid is washing water, disinfecting water, rinsing water, air, or alcohol. In this case, one channel is provided in the insertion portion 102, and actions by the endoscope washing and disinfecting apparatus 1 to wash and disinfect an endoscope 101 including a single channel connector portion 104 as an opening of a channel provided in the operation portion 103 are described.

Further, in a state in which fluid is not being supplied from the inside of the apparatus main body 2 via the fluid supply tube 83, as shown in Fig. 26, in the conduit connection unit 5 the flange 74b of the first nozzle pipe 74 of the nozzle unit 70 receives an urging force from the spring 73, and the first nozzle pipe 74 and second nozzle pipe 75 inside the nozzle housing 51 are urged in the proximal end direction, i.e. towards the rear. At this time, the conduit connector 71 that is connected to the first nozzle pipe 74 is in a state in which the conduit connector 71 is housed inside the recess 72a formed in the base 72.

First, at each washing and disinfecting process to wash and disinfect the endoscope 101, the endoscope washing and disinfecting apparatus 1 moves the conduit connector 71 of the nozzle unit 70 to a position (position A in Fig. 27) that matches the first nozzle outlet opening 65 of the distal end face 61a of the distal end frame 61. That is, by driving and stopping the motor 57 of the conduit connection unit 5 using the control of the control portion 46, the endoscope washing and disinfecting apparatus 1 stops the rotating position of the rotary cylinder 50 at a position the conduit connector 71 and the first nozzle outlet opening 65 are aligned, based on detection signals from the rotational position detecting switch 59.

In this state, when fluid is supplied via the fluid supply tube 83 (see Fig. 26) from inside the apparatus main body 2, as shown in Fig. 30, in the conduit connection unit 5, the resistor 75a of the second nozzle pipe 75 receives the fluid pressure and the first nozzle pipe 74 and the second nozzle pipe 75 move forward in resistance to the urging force of the spring 73. Thus, at the nozzle unit 70, the first nozzle pipe 74 is pushed out to the front so as to lead out from the recess 72a formed in the base 72 along with the conduit connector 71.

More specifically, when fluid from the fluid supply tube 83 is fed to the conduit connection unit 5, a pressure is applied to the resistor 75a of the second nozzle pipe 75. The fluid then flows to a recess 75c formed in the proximal end face of the resistor 75a. Thereupon, as shown in Fig. 26, since the conduit diameter φa of the second nozzle pipe 75 is smaller than the conduit diameter φb of the nozzle insertion hole portion 51a of the nozzle housing 51, when fluid flows into the conduit of the second nozzle pipe 75, the fluid that passes through applies a force to the resistor 75a that pushes the resistor 75a forward.

In this connection, since the pressure applied by the fluid to the resistor 75a must be greater than an urging force from the spring 73, the fluid supply pressure of each pump and the compressor and the urging force of the spring 73 are set to a prescribed pressure or force.

The conduit connector 71 that moves in response to movement of the first nozzle pipe 74 and the second nozzle pipe 75 that are pushed outward upon receiving the pressure of the fluid is led out from inside the recess 72a formed in the base 72 towards the operation portion 103 of the endoscope 101. The conduit connector 71 of the nozzle unit 70 then enters a state in which the conduit connector 71 contacts against the channel connector portion 104 of the operation portion 103 and is connected by the fluid pressure.

At this time, the gasket 76 provided in the conduit connector 71 enters a state of close contact with the opening of the channel connector portion 104. Consequently, fluid flowing inside the first nozzle pipe 74 and the second nozzle pipe 75 is supplied into the channel provided in the endoscope 101 through the channel connector portion 104. Further, when the conduit connector 71 advances and connects with the channel connector portion 104, the tapered surface 7 1 a formed in the front of the conduit connector 71 serves as a guide way so that the opening of the channel connector portion 104 is guided to the center of the conduit connector 71. Thus, even if positioning of the operation portion 103 of the endoscope 101 in the washing bath 4 at a predetermined position defined by the pins 4a is out of alignment to a certain extent, connection of the conduit connector 71 and the channel connector portion 104 is reliably performed.

Further, during each washing and disinfecting process performed by the endoscope washing and disinfecting apparatus 1, the supply of fluid to the channel of the endoscope 101 is temporarily stopped. Thereupon, pressure produced by fluid to the resistor 75a stops, and by receiving the urging force from the spring 73 that contacts against the flange 74b of the first nozzle pipe 74, the first nozzle pipe 74 and the second nozzle pipe 75 are urged towards the proximal end direction, i.e. rearward, and housed inside the nozzle housing 51 of the rotary cylinder 50. At this time, since the conduit connector 71 connected to the first nozzle pipe 74 moves in response to movement of the first nozzle pipe 74 to the proximal end side so as to be housed inside the recess 72a formed in the base 72, the conduit connector 71 releases the connection with the channel connector portion 104.

Next, in the endoscope washing and disinfecting apparatus 1, as shown in Fig. 31, the conduit connector 71 of the nozzle unit 70 moves towards and contacts against the first nozzle abutment portion 68a and the second nozzle abutment portion 68b on the distal end face 61 a of the distal end frame 61 so that movement thereof is controlled at those two positions (positions D and E in Fig. 28). At this time, by driving and stopping the motor 57 of the conduit connection unit 5 by control of the control portion 46, the endoscope washing and disinfecting apparatus 1 rotatingly controls the rotary cylinder 50 during each washing and disinfecting step to move to a position at which the conduit connector 71 and the first nozzle abutment portion 68a contact against each other (position D in Fig. 28) and a position at which the conduit connector 71 and the second nozzle abutment portion 68b contact against each other (position E in Fig. 28), based on detection signals of the rotational position detecting switch 59. Further, the endoscope washing and disinfecting apparatus 1 stops rotation of the rotary cylinder 50 for a predetermined time at each position at which the conduit connector 71 contacts against the first nozzle abutment portion 68a and the second nozzle abutment portion 68b.

In this connection, these two positions are positions at which, by rotation of the rotary cylinder 50 from a position at which the conduit connector 71 of the nozzle unit 70 and the first nozzle outlet opening 65 are aligned, the nozzle unit 70 is not on the same axis as and deviates to some extent by a predetermined distance with respect to each of the channel connector portions 104 arranged in the operation portion 103 of the endoscope 101. That is, these two positions are two positions that deviate by a prescribed angle, which is 45° according to the present embodiment, in the clockwise direction and counterclockwise direction, respectively, around the central axis O of the rotary cylinder 50 from the position at which the nozzle unit 70 and the channel connector portion 104 connect.

In a state in which the rotary cylinder 50 has been stopped at these two positions, the endoscope washing and disinfecting apparatus 1 supplies a fluid to the conduit connection unit 5. In this case, the fluid is washing water, disinfecting water, rinsing water, air, or alcohol. At this time, since the conduit connector 71 abuts against the first nozzle abutment portion 68a or the second nozzle abutment portion 68b, forward movement of the nozzle unit 70 is regulated. That is, even when fluid is fed into the conduit connection unit 5 and pressure is applied to the resistor 75a of the second nozzle pipe 75, the nozzle unit 70 is not led out from the nozzle housing 51.

Thereupon, as shown in Fig. 32, fluid is ejected from the conduit connector 71 of the nozzle unit 70 at a position that deviates from and is not on the same axis as the channel connector portion 104 provided in the operation portion 103 of the endoscope 101. Thus, the fluid from the conduit connector 71 is ejected towards the outer surface and root portion of the channel connector portion 104.

After the lapse of a predetermined time, the endoscope washing and disinfecting apparatus 1 stops the supply of fluid to the conduit connection unit 5 and rotatingly controls the rotary cylinder 50 so that the conduit connector 71 of the nozzle unit 70 stops at a position that contacts against the second nozzle abutment portion 68b. The endoscope washing and disinfecting apparatus 1 then supplies fluid to the conduit connection unit 5 for a predetermined time.

At this time also, as described above, fluid is ejected from the conduit connector 71 of the nozzle unit 70 at a position that deviates from and is not on the same axis as the channel connector portion 104 provided in the operation portion 103. Thus, the fluid from the conduit connector 71 is ejected towards the outer surface and the root portion of the channel connector portion 104.

The endoscope washing and disinfecting apparatus 1 executes the series of operations described above for each process that washes and disinfects the endoscope 101. In this manner the endoscope washing and disinfecting apparatus 1 can wash and disinfect the outer surface and the root portion of the channel connector portion 104 at two positions that deviate from the position of the channel connector portion 104 of the endoscope 101.

As described above, the endoscope washing and disinfecting apparatus 1 of the present embodiment is configured such that, since the endoscope washing and disinfecting apparatus 1 supplies a fluid such as rinsing water, washing water, disinfecting water, alcohol, or air into a channel of the endoscope 101, the nozzle unit 70 that is mainly constituted by the conduit connector 71, the first nozzle pipe 74, and the second nozzle pipe 75 is caused to advance and connect to the channel connector portion 104 by the pressure of the fluid flowing inside the apparatus.

Further, the endoscope washing and disinfecting apparatus 1 is capable of ejecting fluid from the nozzle unit 70 towards the outer surface and the root portion of the channel connector portion 104 and can effectively execute various washing and disinfecting processes including washing and disinfecting the channel connector portion 104 and flushing with alcohol and drying in a short time. That is, when the nozzle unit 70 is not connected to a channel connector portion 104, the endoscope washing and disinfecting apparatus 1 moves to two positions that deviate from the position of the channel connector portion 104 to execute a washing and disinfecting process by ejecting fluid towards the outer surface and the root portion of the channel connector portion 104.

Therefore, in the endoscope washing and disinfecting apparatus 1, even when an opening end portion of the channel connector portion 104 is, for example as shown in Fig. 30 and Fig. 32, an outward flange shape, the ejected fluid splashes back from the surface of the operation portion 103 so that the rear surface side of the outward flange shape of the opening end portion described above can be easily washed and disinfected.

As described above, during a process to wash and disinfect the inside of a channel of the endoscope 101, the endoscope washing and disinfecting apparatus 1 of the present embodiment directly ejects a fluid such as a cleaning fluid or a disinfecting fluid from two positions that deviate from and are not on the same axis as the channel connector portion 104 that is the opening of the channel, and hence the endoscope washing and disinfecting apparatus 1 can effectively wash and disinfect the outer surface and the root portion of the channel connector portion 104. As a result, the time required for the process of washing and disinfecting the outer surface and the root portion of the channel connector portion 104 can be reduced.

Next, using Figs. 33 and 34, a case is briefly described in which the endoscope washing and disinfecting apparatus 1 washes and disinfects an endoscope 101 having two channels inside the insertion portion 102 and in which two channel connector portions 104 are provided in the operation portion 103 in correspondence to the two channels.

The endoscope washing and disinfecting apparatus 1 recognizes that there are two channel connector portions 104 provided in the operation portion 103 based on model information (the endoscope ID) of the endoscope 101 by reading an RFID of the endoscope 101 to be washed or by manual input of the model information by the user, and executes driving control of the conduit connection unit 5 and the washing and disinfecting process using the control portion 46.

As shown in Fig. 33, at a position (position B in Fig. 27) at which the conduit connector 71 of the nozzle unit 70 is aligned with the second nozzle outlet opening 66 of the opening 64 formed in the distal end face 61a of the distal end frame 61, one of the two channel connector portions 104 of the endoscope 101 and the nozzle unit 70 are positioned on the same axis. That is, at this position, when fluid is supplied to the conduit connection unit 5, the nozzle unit 70 advances and the conduit connector 71 connects with one of the channel connector portions 104.

The endoscope washing and disinfecting apparatus 1 continues to supply a fluid, in this case, washing water, disinfecting water, rinsing water, air, or alcohol, to the conduit connection unit 5 for a predetermined time during each washing and disinfecting process to connect the conduit connector 71 of the nozzle unit 70 and one of the channel connector portions 104 and thereby wash and disinfect the inside of one of the channels of the endoscope 101.

Further, the endoscope washing and disinfecting apparatus 1 temporarily stops the fluid supply to the conduit connection unit 5, and controls rotation of the rotary cylinder 50 around the central axis O in the clockwise direction and counterclockwise direction at a prescribed angle, which is 45°, respectively, according to the present embodiment, to arrive at two positions (positions D and F in Fig. 28) from the position at which the conduit connector 71 of the nozzle unit 70 is aligned with the second nozzle outlet opening 66 of the opening 64. At this time, the conduit connector 71 contacts against the first nozzle abutment portion 68a or the third nozzle abutment portion, so that forward movement of the nozzle unit 70 is regulated and the nozzle unit 70 is not led out from the nozzle housing 51.

In these two states, the endoscope washing and disinfecting apparatus 1 again supplies fluid to the conduit connection unit 5 to eject the fluid from the conduit connector 71. That is, fluid is ejected from the conduit connector 71 of the nozzle unit 70 at a position that is not on the same axis as the relevant channel connector portion 104 provided in the operation portion 103. Thus, the fluid from the conduit connector 71 is ejected towards the outer surface and the root portion of one of the channel connector portions 104.

Further, as shown in Fig. 34, at a position (position C in Fig. 27) at which the conduit connector 71 of the nozzle unit 70 is aligned with the third nozzle outlet opening 67 of the opening 64 formed in the distal end face 61 a of the distal end frame 61, the other of the two channel connector portions 104 of the endoscope 101 and the nozzle unit 70 are positioned on the same axis. That is, at this position, when fluid is supplied to the conduit connection unit 5, the nozzle unit 70 advances and the conduit connector 71 connects with the other of the channel connector portions 104.

Similarly to the case of washing and disinfecting the first channel connector portion 104 described above, the endoscope washing and disinfecting apparatus 1 continues to supply the fluid to the conduit connection unit 5 for a predetermined time during each washing and disinfecting process to connect the conduit connector 71 of the nozzle unit 70 to the other channel connector portion 104 and thereby wash and disinfect the inside of the other channel of the endoscope 101.

Further, the endoscope washing and disinfecting apparatus 1 temporarily stops the fluid supply to the conduit connection unit 5, and controls rotation of the rotary cylinder 50 around the central axis O in the clockwise direction and counterclockwise direction at a prescribed angle, which is 45°, respectively, according to the present embodiment, to arrive at two positions (positions E and G in Fig. 28) from the position at which the conduit connector 71 of the nozzle unit 70 is aligned with the third nozzle outlet opening 67 of the opening 64. At this time, the conduit connector 71 contacts against the second nozzle abutment portion 68a or the third nozzle abutment portion 68c, so that forward movement of the nozzle unit 70 is regulated and the nozzle unit 70 is not led out from the nozzle housing 51.

In these two states also, the endoscope washing and disinfecting apparatus 1 again supplies fluid to the conduit connection unit 5 to eject the fluid from the conduit connector 71. That is, fluid is ejected from the conduit connector 71 of the nozzle unit 70 at two positions that are not on the same axis as the relevant channel connector portion 104 provided in the operation portion 103. Thus, the fluid from the conduit connector 71 is ejected towards the outer surface and the root portion of one of the channel connector portions 104.

The endoscope washing and disinfecting apparatus 1 executes the series of operations described above for each process that washes and disinfects the endoscope 101. In this manner the endoscope washing and disinfecting apparatus 1 can wash and disinfect the outer surface and the root portion of the channel connector portions 104 at two positions that deviate from the positions of the channel connector portions 104 of the endoscope 101, even when the endoscope 101 has two channel connector portions 104.

It should be understood that the invention described above is not limited to the above described embodiments, and various modifications can be made thereto at the execution stage without departing from the spirit or scope of the invention. Further, inventions of various stages are included in the embodiments and various inventions can be extracted by suitability combining a plurality of the configurational requirements that are disclosed.

For example, in a case in which the problem set forth as a problem to be solved by the invention can be solved and the effect described as the effect of the invention can be obtained even if several of the configurational requirements are deleted from the entire configurational requirements disclosed in an embodiment, the configuration from which the relevant configurational requirements were deleted can be extracted as an invention.

Having described the preferred embodiments of the invention referring to the accompanying drawings, it should be understood that the present invention is not limited to those precise embodiments and various changes and modifications thereof could be made by one skilled in the art without departing from the spirit or scope of the invention as defined in the appended claims.

## Claims

1. An endoscope washing and disinfecting apparatus, comprising:
an apparatus main body including a washing bath in which an endoscope is mounted;
a nozzle unit that is arranged so as to face a conduit connector portion of an endoscope conduit that is provided in the endoscope mounted in the washing bath, to which fluid within the apparatus main body is supplied so that the nozzle unit advances due to pressure of the fluid, and which supplies the fluid to inside the endoscope conduit by connecting to the conduit connector portion; and
an automatic brush mechanism that is arranged in the apparatus main body and that carries out brushing and cleaning within the endoscope conduit by introducing a cleaning brush into the endoscope conduit through the nozzle unit and moving the cleaning brush forward and rearward.

2. The endoscope washing and disinfecting apparatus according to claim 1, further comprising:
a rotary member in which the nozzle unit is arranged at a decentered position, and which rotates taking a center thereof as an axis;
a driving source that rotates the rotating member; and
a control portion that drives and stops the driving source to control a rotating position of the rotating member to reach a position at which the nozzle unit is connected to the conduit connector portion.

3. The endoscope washing and disinfecting apparatus according to claim 2,
wherein, based on specific information of the endoscope, the control portion determines whether there is one conduit connector portion or a plurality of conduit connector portions, and drives and stops the driving source to control a rotating position of the rotating member so that the nozzle unit moves to a connecting position in correspondence with one or a plurality of the conduit connector portions.

4. The endoscope washing and disinfecting apparatus according to claim 3,
wherein, the nozzle unit is moved to and stopped at a position on a same axis as the one conduit connector portion or the plurality of conduit connector portions, and advances due to pressure of the fluid to connect to the conduit connector portion.

5. The endoscope washing and disinfecting apparatus according to any one of claims 2 to 4, further comprising:
a regulating portion that is provided at a position that deviates at a prescribed angle from a position at which the nozzle unit that is rotatingly moved by rotation of the rotating member is connected with the conduit connector portion, and which abuts against and regulates forward movement of the nozzle unit;
wherein the control portion drives and stops the driving source to control a rotating position of the rotating member to reach a position at which the nozzle unit is connected to the conduit connector portion and a position at which the nozzle unit is regulated by the regulating portion.

6. The endoscope washing and disinfecting apparatus according to any one of claims 2 to 5, further comprising:
a detection portion that detects a rotating position of the rotating member and outputs a detection signal to the control portion.

7. The endoscope washing and disinfecting apparatus according to claim 5 or claim 6,
wherein the regulating portion is provided at two positions which deviate by the prescribed angle in a clockwise direction and a counterclockwise direction with respect to a center axis of the rotating member from a position at which the nozzle unit is connected to the conduit connector portion.
